(19) Europäisches Patentamt
European Patent Office
Office européen des brevets

(11) EP 1 223 209 B1

(12) EUROPÄISCHE PATENTSCHRIFT

(45) Veröffentlichungstag und Bekanntmachung des Hinweises auf die Patenterteilung:
**03.03.2004 Patentblatt 2004/10**

(51) Int Cl.[7]: **C09K 19/32**, C09K 19/42, C07C 25/00, C07C 43/225

(21) Anmeldenummer: **02000192.1**

(22) Anmeldetag: **10.01.2002**

(54) **Fluorierte Fluorene und ihre Verwendung in Flüssigkristallmischungen**

Fluorinated fluorenes and use thereof in liquid crystal mixtures

Fluorènes fluorés et leur utilisation dans des mélanges liquides cristallins

(84) Benannte Vertragsstaaten:
**DE FR GB**

(30) Priorität: **11.01.2001 DE 10101020**

(43) Veröffentlichungstag der Anmeldung:
**17.07.2002 Patentblatt 2002/29**

(73) Patentinhaber: **CLARIANT INTERNATIONAL LTD.
4132 Muttenz (CH)**

(72) Erfinder:
- **Schmidt, Wolfgang Dr.
63303 Dreieich (DE)**
- **Wingen, Rainer Dr.
65795 Hattersheim (DE)**
- **Hornung, Barbara Dl.
63594 Hasselroth (DE)**

(74) Vertreter: **Isenbruck, Günter, Dr. et al
Isenbruck, Bösl, Hörschler, Wichmann, Huhn, Patentanwälte
Theodor-Heuss-Anlage 12
68165 Mannheim (DE)**

(56) Entgegenhaltungen:
**EP-A- 0 311 010**
**WO-A-01/10803**
**DE-A- 2 145 650**
**GB-A- 1 274 152**

- **TOMIOKA, HIDEO ET AL: "Flash vacuum pyrolysis of trifluoromethylated diphenyldiazomethanes. Role of trifluoromethyl group in the arylcarbene rearrangement" CHEM. COMMUN. (CAMBRIDGE) (1997), (18), 1745-1746 , XP002196883**
- **DATABASE CA [Online] CHEMICAL ABSTRACTS SERVICE, COLUMBUS, OHIO, US; YANO, HITOSHI ET AL: "Liquid-crystal compound with high negative dielectric anisotropy, its composition, and display device using it" retrieved from STN Database accession no. 130:304122 XP002196881 & JP 10 236992 A (CHISSO CORP., JAPAN) 8. September 1998 (1998-09-08)**
- **DATABASE CA [Online] CHEMICAL ABSTRACTS SERVICE, COLUMBUS, OHIO, US; SHUNDO, RYUJI ET AL: "Fluorene derivative compound and liquid crystal composition containing it" retrieved from STN Database accession no. 133:66049 XP002196880 & JP 2000 178211 A (CHISSO CORP., JAPAN) 27. Juni 2000 (2000-06-27)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 223 209 B1

Printed by Jouve, 75001 PARIS (FR)

## Beschreibung

Viele Hersteller haben elektrooptische Anzeigen auf der Basis nematischer Flüssigkristalle entwickelt, die seit einigen Jahren beispielsweise im Bereich von Notebook PCs, Personal Digital Assistants und Desktop Monitoren im Einsatz sind. Dabei werden die Technologien TN (Twisted Nematics), STN (Supertwisted Nematics), AM-TN (Active Matrix - Twisted Nematics), AM-IPS (Active Matrix - In Plane Switching), AM-MVA (Active Matrix - Multidomain Vertically Aligned) verwendet, die in der Literatur ausführlich beschrieben werden, siehe z. B. T. Tsukuda, TFT/LCD: Liquid Crystal Displays Addressed by Thin-Film Transistors, Gordon and Breach 1996, ISBN 2-919875-01-9 und darin zitierte Literatur; SID Symposium 1997, ISSN-0097-966X, Seiten 7 bis 10, 15 bis 18, 47 bis 51, 213 bis 216, 383 bis 386, 397 bis 404 und darin zitierte Literatur.

Clark und Lagerwall (US 4,367,924) konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN (Twisted Nematic)-Zellen um bis zu einen Faktor 1000 schnellere Schaltzeiten haben (siehe z. B. EP-A-0 032 362). Aufgrund dieser und anderer günstiger Eigenschaften, z. B. der bistabilen Schaltmöglichkeit und des nahezu blickwinkelunabhängigen Kontrasts, sind FLCs grundsätzlich für Anwendungsgebiete wie Computerdisplays und Fernsehgeräte geeignet, wie ein seit Mai 1995 in Japan von Canon vermarkteter Desktop-Monitor zeigt.

Die einzelnen Bildelemente (Pixel) eines LC-Displays sind üblicherweise in einer x,y-Matrix angeordnet, die durch die Anordnung je einer Serie von Elektroden (Leiterbahnen) entlang der Reihen und der Spalten an der Unter- bzw. Oberseite des Displays gebildet wird. Die Kreuzungspunkte der horizontalen (Reihen-) und vertikalen (Spalten-) Elektroden bilden adressierbare Pixel.

Diese Anordnung der Bildpunkte bezeichnet man üblicherweise als eine passive Matrix. Zur Adressierung wurden verschiedene Multiplex-Schemata entwickelt, wie beispielsweise in Displays 1993, Vol. 14, Nr. 2, S. 86-93 und Kontakte 1993 (2), S. 3-14 beschrieben. Die passive Matrixadressierung hat den Vorteil einer einfacheren Herstellung des Displays und damit verbundenen geringen Herstellkosten, jedoch den Nachteil, daß die passive Adressierung immer nur zeilenweise erfolgen kann, was dazu führt, daß die Adressierungszeit des gesamten Bildschirms bei N Zeilen das N-fache der Zeilenadressierungszeit beträgt.

Bei der sogenannten Aktivmatrix-Technologie (AM-LCD) wird üblicherweise ein nichtstrukturiertes Substrat mit einem Aktivmatrix-Substrat kombiniert. An jedem Pixel des Aktivmatrixsubstrates ist ein elektrisch nichtlineares Element, beispielsweise ein Dünnschichttransistor, integriert. Bei dem nichtlinearen Element kann es sich auch um Dioden, Metall-Insulator-Metall u.ä. Elemente handeln, die vorteilhaft mit Dünnschichtverfahren hergestellt werden und in der einschlägigen Literatur beschrieben sind, siehe z. B. T. Tsukuda, TFT/LCD: Liquid Crystal Displays Addressed by Thin-Film Transistors, Gordon and Breach 1996, ISBN 2-919875-01-9 und darin zitierte Literatur.

Aktivmatrix-LCDs werden üblicherweise mit nematischen Flüssigkristallen im TN-(Twisted Nematics), ECB-(Electrically Controlled Birefringence), VA- (Vertically Aligned) oder IPS- (In Plane Switching) Modus betrieben. In jedem Fall wird durch die aktive Matrix an jedem Bildpunkt ein elektrisches Feld individueller Stärke erzeugt, das eine Orientierungsänderung und damit eine Änderung der Doppelbrechung erzeugt, die wiederum im polarisierten Licht sichtbar ist. Ein Nachteil dieser Verfahren ist derzeit noch die mangelnde Videofähigkeit bedingt durch die langen Schaltzeiten nematischer Flüssigkristalle.

Unter anderem aus diesem Grunde wurden auch Flüssigkristallanzeigen, die auf einer Kombination aus ferroelektrischen Flüssigkristallmaterialien und aktiven Matrix-Elementen beruhen,vorgeschlagen, siehe z. B. WO 97/12355, Ferroelectrics 1996, 179, 141-152, oder WO 99/60441.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses ist eine einheitliche planare Orientierung der Flüssigkristalle nötig. Eine gute Orientierung in der $S_A$ und $S^*_C$-Phase läßt sich z. B. erreichen, wenn die Phasenfolge der Flüssigkristallmischung mit abnehmender Temperatur lautet:

$$\text{Isotrop} \rightarrow N^* \rightarrow S_A \rightarrow S^*_C$$

Voraussetzung ist, daß der Pitch (Ganghöhe der Helix) in der N*-Phase sehr groß (größer 10 µm) oder, noch besser, völlig kompensiert ist (siehe z. B. T. Matsumoto et al., Proc. of the 6th Int. Display Research Conf., Japan Display, Sept. 30 - Okt. 2, 1986, Tokyo, Japan, S. 468-470; M. Murakami et al., ibid. S. 344-347). Dies erreicht man z. B., indern man zu der chiralen Flüssigkristallmischung, die in der N*-Phase z. B. eine linksdrehende Helix aufweist, einen oder mehrere optisch aktive Dotierstoffe, die eine rechtsdrehende Helix induzieren, in solchen Mengen hinzugibt,

daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C*-Phase wesentlich größer ist als die Dicke des Anzeigeelementes (Mol. Cryst. Liq. Cryst. 1983, 94, 213 und ibid. 1984, 114, 151).

Die optische Schaltzeit $\tau$ [µs] ferroelektrischer Flüssigkristallsysteme, die möglichst kurz sein soll, hängt von der Rotationsviskosität des Systems $\gamma$ [mPas], der spontanen Polarisation Ps [nC/cm$^2$] und der elektrischen Feldstärke E [V/m] ab nach der Beziehung

$$\tau \sim \frac{\gamma}{Ps \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und/oder eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$ und eine geringe positive oder vorzugsweise negative dielektrische Anisotropie $\Delta\varepsilon$ verlangt (siehe z. B. S. T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktsemiedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll. Zudem ist es notwendig, daß die unterschiedlichen Komponenten in einem breiten Temperaturbereich untereinander gut mischbar sind.

Da aber die Entwicklung von Flüssigkristallmischungen noch in keiner Weise als abgeschlossen betrachtet werden kann, sind die Hersteller von Displays an den unterschiedlichsten Komponenten für Mischungen interessiert.

Insbesondere werden Flüssigkristallmischungen benötigt, die einerseits einen sehr weiten Arbeits-Temperaturbereich aufweisen, andererseits eine möglichst geringe Schwellspannung besitzen, beispielsweise für den Einsatz in Automobilen, in denen ohne weiteres ein Temperaturbereich von -40°C bis 100°C auftreten kann, aber auch für tragbare Geräte wie Mobiltelefone und Notebook-PCs.

Darüber hinaus scheint der Trend hin zu bewegten Bildern mit nematischen Flüssigkristallen nur dann realisierbar zu sein, wenn man den Elektrodenabstand solcher LCDs verringert, denn die Schaltzeiten sind umgekehrt proportional zum Quadrat des Elektrodenabstandes. Dies wiederum zieht unweigerlich eine Verringerung von Helligkeit und Kontrast nach sich, es sei denn, man verwendet Flüssigkristallmischungen mit einem hohen Wert der optischen Anisotropie ($\Delta n$). Gewöhnlich besitzen Materialien mit hoher optischer Anisotropie jedoch auch hohe Viskositäten und wirken daher dem gesetzten Ziel entgegen. Es besteht daher Nachfrage nach neuen, geeigneten Flüssigkristallmischungen und -mischungskomponenten, die einen hohen Klärpunkt und eine hohe optische Anisotropie besitzen, bei gleichzeitig verhältnismäßig geringer Rotationsviskosität.

Aufgabe der vorliegenden Erfindung ist es daher, neue Komponenten für die Verwendung in smektischen oder nematischen Flüssigkristallmischungen bereit zu stellen, die über hohe positive Werte der dielektrischen Anisotropie kombiniert mit einem günstigen Verhältnis von Viskosität und Klärpunkt verfügen. Darüber hinaus sollen die Verbindungen in hohem Maße licht- und UV-stabil sowie thermisch stabil sein. Ferner sollen sie geeignet sein, eine hohe "voltage holding ratio (VHR)" zu realisieren. Außerdem sollten sie synthetisch gut zugänglich und daher potentiell kostengünstig sein.

Für die Verwendung in flüssigkristallinen Mischungen sind Derivate des Fluorens und Fluorenons bereits aus J. Chem. Soc. 1955, 4359, ibid. 1957, 3228, Pramana Suppl. 1975, 1, 397, Bull. Soc. Chim. Fr. 1975, 2066, Mol. Cryst. Liq. Cryst 1985, 127, 341, ibid. 1985, 129, 17, ibid. 1987, 150B, 361, ibid. 1988, 164, 167, ibid. 1990, 182, 339, EP-A 0 113 293, EP-B 0 325 035, JP 05 125055, JP 2000 178211 und EP-A 0 455 219 bekannt.

In der JP 10 236992 läßt sich aus der dort angegebenen, allgemeinen Formel unter einer Vielzahl anderer Möglichkeiten auch ein 2,7-disubstituiertes 3,4,5,6-Tetrafluorfluorenderivat konstruieren, in der Anmeldung wird jedoch auf die Synthese oder physikalische Eigenschaften entsprechender Verbindungen nicht eingegangen.

1-Fluorfluoren-9-on ist beispielsweise aus Chem. Ind. 1961, 179-180 und weitere, mehrfach fluorierte Fluorenderivate beispielsweise aus Can. J. Chem. 1967, 45, 2569-2575 bekannt. Eine Eignung dieser Moleküle oder deren Derivate als Teil einer Komponente von Flüssigkristallmischungen läßt sich für den Fachmann daraus jedoch nicht ableiten.

Es wurde nun gefunden, daß Verbindungen der Formel (I)

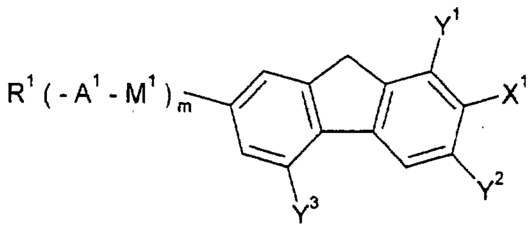

(I)

mit den Bedeutungen:

$Y^1$, $Y^2$, $Y^3$ sind gleich oder verschieden H oder F, mit der Maßgabe, daß mindestens einer von $Y^1$, $Y^2$ gleich F sein muß,

$X^1$ ist gleich F, Cl, CN, $CF_3$, $CHF_2$, $CH_2F$, $OCF_3$, $OCHF_2$, $OCH_2F$, $OCH_2CF_3$, $OCH{=}CF_2$ oder die Gruppierung ($M^2$ - $A^2$ -$)_n$ $X^2$,

$X^2$ ist gleich F, Cl, CN, $CF_3$, $CHF_2$, $CH_2F$, $OCF_3$, $OCHF_2$, $OCH_2F$, $OCH_2CF_3$, $OCH{=}CF_2$ oder die Gruppierung $R^2$,

$R^1$, $R^2$ sind gleich oder verschieden
Wasserstoff oder ein geradkettiger oder verzweigter Alkylrest mit 1 bis 16 C-Atomen oder ein geradkettiger oder verzweigter Alkenylrest mit 2 bis 16 C-Atomen, worin

a) eine oder mehrere nicht benachbarte und nicht terminale $CH_2$-Gruppen durch -O-, -C(=O)O-, -O-C(=O)-, -O-C(=O)-O-, -C(=O)- oder -Si($CH_3$)$_2$- ersetzt sein können, und/oder
b) eine $CH_2$-Gruppe durch -C≡C-, Cyclopropan-1,2-diyl oder Cyclobutan-1,3-diyl ersetzt sein kann, und/oder
c) ein oder mehrere H-Atome durch F oder Cl ersetzt sein können, und/oder
d) die terminale $CH_3$-Gruppe durch eine der folgenden chiralen Gruppen (optisch aktiv oder racemisch) ersetzt sein kann:

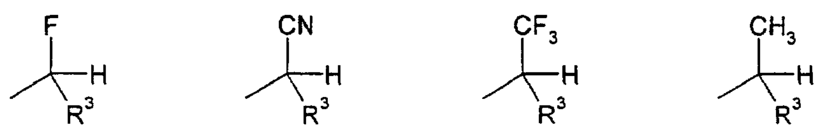

wobei $R^3$ ein geradkettiger oder verzweigter Alkylrest mit 1 bis 12 C-Atomen oder ein geradkettiger oder verzweigter Alkenylrest mit 2 bis 12 C-Atomen ist, worin eine oder mehrere nicht benachbarte und nicht terminale $CH_2$-Gruppen durch -O- ersetzt sein können und/oder ein oder mehrere H-Atome durch F oder Cl ersetzt sein können,

mit der Maßgabe, daß nur einer von $R^1$, $R^2$ gleich Wasserstoff sein kann,

$M^1$, $M^2$ sind gleich oder verschieden
-C(=O)-O-, -O-C(=O)-, -$CH_2$-O-, -O-$CH_2$-, -$CF_2$-O-, -O-$CF_2$-, -CH=CH-, -CF=CF-, -C≡C-, -$CH_2$-$CH_2$-C(=O)-O-, -O-C(=O)-$CH_2$-$CH_2$-, -$CH_2$-$CH_2$-, -$CF_2$-$CF_2$-, -($CH_2$)$_4$- oder eine Einfachbindung,

$A^1$, $A^2$ sind gleich oder verschieden
1,4-Phenylen, worin ein oder zwei H-Atome durch F, Cl oder CN ersetzt sein können, Pyridin-2,5-diyl, worin ein H-Atom durch F ersetzt sein kann, Pyrimidin-2,5-diyl, 1,4-Cyclohexylen, worin ein oder zwei H-Atome durch $CH_3$ oder F ersetzt sein können, 1-Cyclohexen-1,4-diyl, worin ein H-Atom durch $CH_3$ oder F ersetzt sein kann, oder 1,3-Dioxan-2,5-diyl,

m, n sind unabhängig voneinander 0 oder 1, in Summe jedoch nicht größer als 1,

diesen Anforderungen in besonderer Weise genügen.

Liegen an mehreren der genannten Positionen Ersetzungen vor, so sind sie voneinander unabhängig aus den genannten Ersetzungen ausgewählt und können daher gleich oder verschieden sein. Es können damit in der Verbindung auch mehrere unterschiedliche der genannten Ersetzungen an unterschiedlichen Positionen gleichzeitig vorliegen.

Bevorzugt sind die Verbindungen der Formel (I) mit den Bedeutungen:

$X^1$ ist gleich F, $OCF_3$ oder die Gruppierung $(M^2 - A^2 -)_n X^2$,

$X^2$ ist gleich F, $OCF_3$ oder die Gruppierung $R^2$,

$R^1$, $R^2$ sind gleich oder verschieden
ein geradkettiger oder verzweigter Alkylrest mit 1 bis 14 C-Atomen oder ein geradkettiger oder verzweigter Alkenylrest mit 2 bis 14 C-Atomen, worin eine oder zwei nicht benachbarte und nicht terminale $CH_2$-Gruppen durch -O- ersetzt sein können und/oder ein oder mehrere H-Atome durch F ersetzt sein können,

$R^3$ ist ein geradkettiger oder verzweigter Alkylrest mit 1 bis 10 C-Atomen, worin eine nicht terminale $CH_2$-Gruppe durch -O- ersetzt sein kann,

$M^1$, $M^2$ sind gleich oder verschieden
$-CH_2-O-$, $-O-CH_2-$, $-CH_2-CH_2-$ oder eine Einfachbindung,

$A^1$, $A^2$ sind gleich oder verschieden
1,4-Phenylen, worin ein oder zwei H-Atome durch F ersetzt sein können, oder 1,4-Cyclohexylen,

m, n sind unabhängig voneinander 0 oder 1, in Summe jedoch nicht größer als 1.

Besonders bevorzugt sind die folgenden Verbindungen der Formeln (I a) bis (I k)

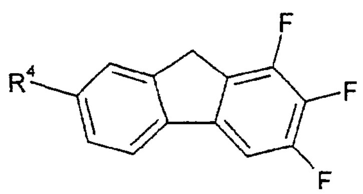
(I a)

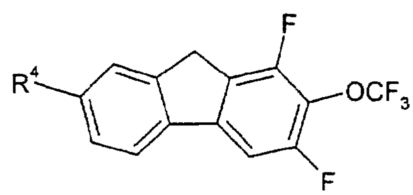
(I b)

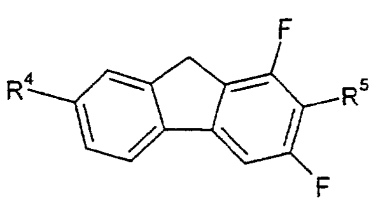
(I c)

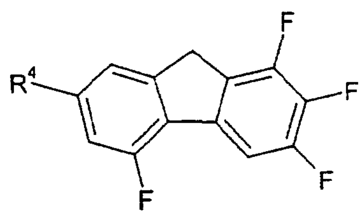
(I d)

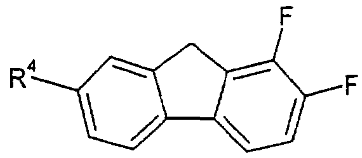

(I e)

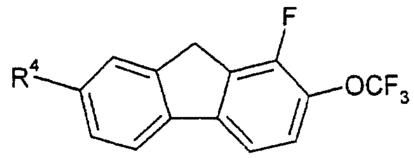

(I f)

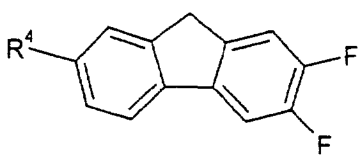

(I g)

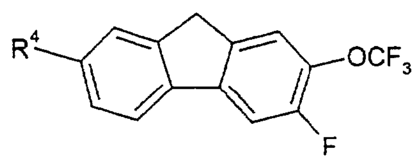

(I h)

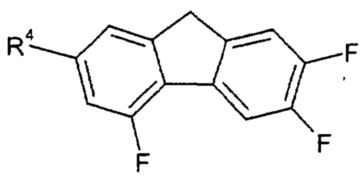

(I i)

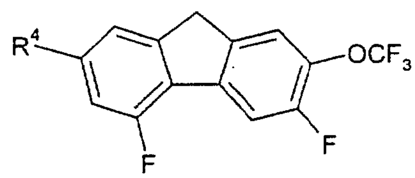

(I k)

mit den Bedeutungen:

$R^4$ ist ein geradkettiger Alkyl-, Alkenyl- oder Alkoxyrest mit 2 bis 12 C-Atomen, worin auch die direkt mit der Fluoreneinheit verknüpfte $CH_2$-Gruppe durch 1,4-Cyclohexylen oder 1,4-Phenylen, welches gegebenenfalls mit ein oder zwei F substituiert ist, ersetzt sein kann,

$R^5$ ist gleich H oder ein geradkettiger Alkyl-, Alkenyl- oder Alkoxyrest mit 2 bis 12 C-Atomen, worin auch die direkt mit der Fluoreneinheit verknüpfte $CH_2$-Gruppe durch 1,4-Cyclohexylen oder 1,4-Phenylen, welches gegebenenfalls mit ein oder zwei F substituiert ist, ersetzt sein kann,

mit der Maßgabe, daß nur in einer von $R^4$, $R^5$ die direkt mit der Fluoreneinheit verknüpfte $CH_2$-Gruppe durch 1,4-Cyclohexylen oder 1,4-Phenylen, welches gegebenenfalls mit ein oder zwei F substituiert ist, ersetzt sein kann.

Ganz besonders bevorzugt sind Verbindungen der Formeln (I a) bis (I f) mit den Bedeutungen:

$R^4$ ist ein geradkettiger Alkyl-, Alkenyl- oder Alkoxyrest mit 2 bis 10 C-Atomen, worin auch die direkt mit der Fluoreneinheit verknüpfte $CH_2$-Gruppe durch 1,4-Cyclohexylen oder 1,4-Phenylen ersetzt sein kann,

$R^5$ ist Wasserstoff oder ein geradkettiger Alkyl-, Alkenyl- oder Alkoxyrest mit 2 bis 10 C-Atomen.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich erweitert.

In diesem Zusammenhang besitzen die Verbindungen der Formel (I) einen breiten Anwendungsbereich. In Abhängigkeit von der Auswahl der Substituenten können sie anderen Verbindungsklassen zugesetzt werden, um beispielsweise die dielektrische und/oder optische Anisotropie eines solchen Dielektrikums zu beeinflussen. Auch können sie dazu dienen, dessen Schwellenspannung und/oder dessen Viskosität zu optimieren.

Besonders geeignet sind die Verbindungen der Formel (I), um schon in geringen Zumischmengen die dielektrische Anisotropie ($\Delta\varepsilon$) zu beeinflussen.

Gegenstand der Erfindung ist somit neben Verbindungen der Formel (I) auch die Verwendung dieser Verbindungen als Komponenten flüssigkristalliner Mischungen.

Die Verbindungen der Formel (I) können in Flüssigkristallmischungen eingesetzt werden. Sie eignen sich im Falle von nematischen Mischungen besonders für "Active Matrix Displays" (AM-LCD) (siehe z. B. C. Prince, Seminar Lecture Notes, Volume I, p. M-3/3-M-22, SID International Symposium 1997, B. B: Bahadur, Liquid Crystal Applications and Uses, Vol. 1, p. 410, World Scientific Publishing, 1990, E. Lüder, Recent Progress of AMLCD's, Proceedings of the 15$^{th}$ International Display Research Conference , 1995, p. 9-12) und "in-plane-switching Displays" (IPS-LCD), im Falle von smektischen Flüssigkristallmischungen für geneigt smektische (ferroelektrische oder antiferroelektrische) Displays.

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. In diesem Sinne geeignete Mischungskomponenten sind insbesondere in WO 00/36054, DE-A-195 31 165 sowie EP-A-0 893 424 aufgeführt, auf die hiermit ausdrücklich Bezug genommen wird.

Gegenstand der Erfindung sind auch Flüssigkristallmischungen, die mindestens eine Verbindung der Formel (I), vorzugsweise in einer Menge von 1 bis 40 Gew.-%, bezogen auf die Flüssigkristallmischung, enthalten. Vorzugsweise enthalten die Mischungen mindestens 3 weitere Komponenten bzw. (flüssigkristalline) Verbindungen. Gegenstand der Erfindung sind darüber hinaus auch elektrooptische Anzeigeelemente (Displays), die die erfindungsgemäßen Mischungen enthalten.

Bevorzugt sind Displays, die die erfindungsgemäßen nematischen oder smektischen (ferroelektrischen oder antiferroelektrischen) Mischungen in Kombination mit Aktiv-Matrix-Elementen enthalten.

Die erfindungsgemäßen Anzeigeelemente (Displays) sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z. B. aus Glas) sind. Darüber hinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z. B. E. Kaneko, "Liquid Crystal TV Displays: Principles and Applications of Liquid Crystal Displays", KTK Scientific Publishers, 1987).

Beispielhaft sind in den nachfolgenden Schemata 1 bis 9 mögliche Synthesewege zu Verbindungen der Formel (I) angegeben, wobei auch andere Verfahren denkbar und möglich sind.

In den Schemata 1 bis 9 bedeuten $L^1$, $L^2$ unabhängig voneinander H oder F. Folgende Abkürzungen werden verwendet:

| | |
|---|---|
| *n*-BuLi | *n*-Buthyllithium |
| DCC | Dicyclohexylcarbodiimid |
| DEAD | Diethylazodicarboxylat (Azodicarbonsäurediethylester) |
| Diglyme | Diethylenglykoldimethylether |
| DMAP | 4-(Dimethylamino)pyridin |
| DME | Dimethoxyethan |
| DMF | N,N-Dimethylformamid |
| KO*t*Bu | Kalium-*tert*-butylat |
| LiTMP | Lithium-2,2,6,6-tetramethylpiperidid |
| MTBE | *tert*-Butylmethylether |
| 4-TsOH | 4-Toluolsulfonsäure |

Schema 1

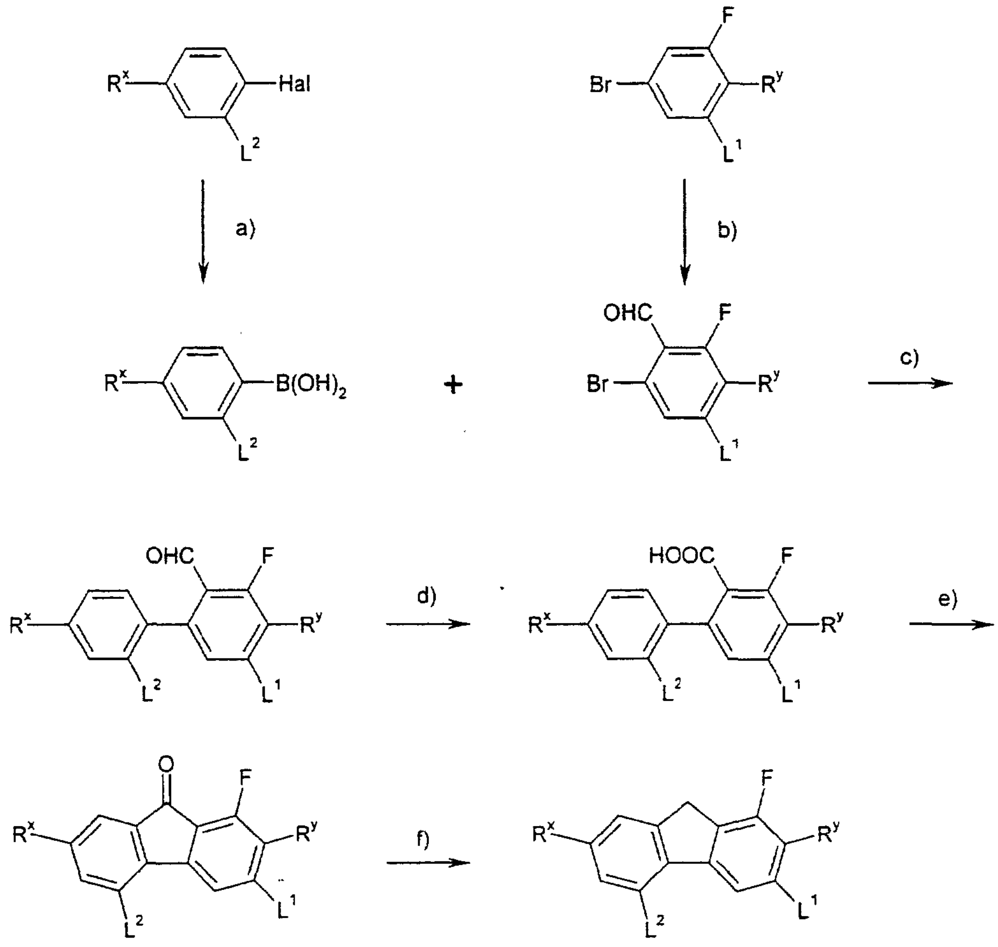


a) 1. Mg/THF 2. $B(OMe)_3$ 3. $H_3O^+$ b) 1. LiTMP/THF/Hexan 2. DMF 3. $H_3O^+$
c) $Pd(PPh_3)_4$/CsF/DME d) $KMnO_4$/Aceton/$H_2O$ e) 1. $SOCl_2$ 2. $AlCl_3$/$CH_2Cl_2$
f) $Et_3SiH$/$F_3CCOOH$

Die in Schema 1 aufgeführten, bei Stufe c) zum Einsatz gelangenden fluorierten 2-Brombenzaldehyde sind in der Literatur beschrieben (2-Brom-6-fluorbenzaldehyd: CAS 59142-68-6; 2-Brom-4,6-difluorbenzaldehyd: CAS 154650-59-6) oder lassen sich aus käuflichem 1-Brom-3,4-difluorbenzol, 1-Brom-3,4,5-trifluorbenzol, 4-Brom-2,6-difluoranisol, 4-Brom-2-fluoranisol oder 1-Brom-3-fluor-4-(trifluormethoxy)benzol durch Metallierung mit Lithium-2,2,6,6-tetramethylpiperidid, Umsetzung mit DMF und Hydrolyse erhalten (siehe Schema 1, Beispiel 1 sowie die Deutsche Patentanmeldung Nr. 10022661,2).

Schema 2

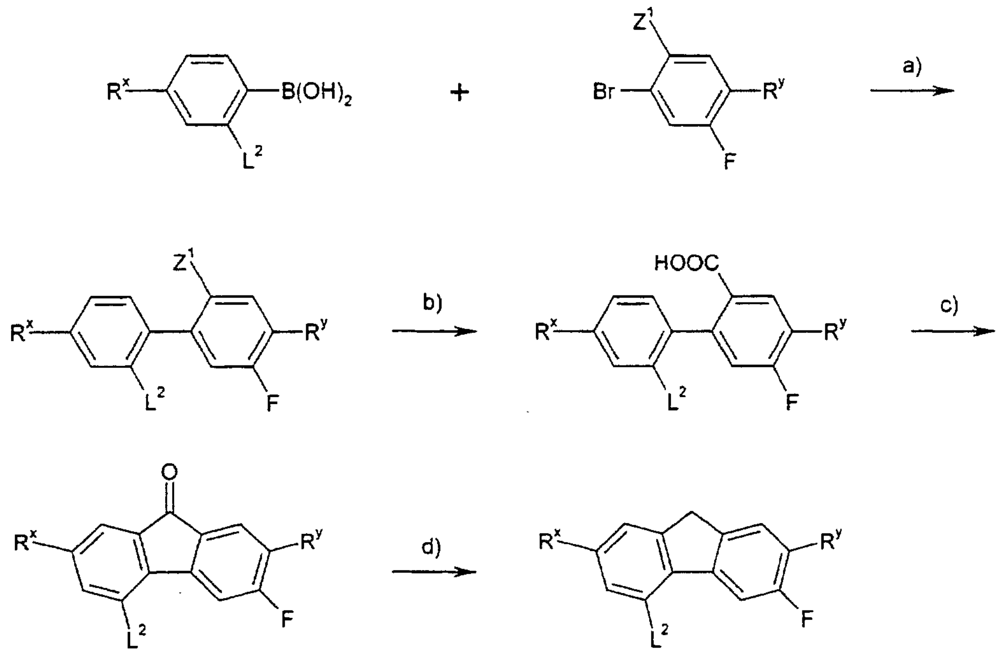


$Z^1$ = CHO, CN, COOH

a) $Pd(PPh_3)_4/CsF/DME$ ($Z^1$ = CHO) bzw. $Pd(PPh_3)_4/Na_2CO_3$/Toluol/EtOH/$H_2O$ ($Z^1$ = CN)

bzw. $Pd(PPh_3)_4/Na_2CO_3/CH_3CN/H_2O$ ($Z^1$ = COOH)

b) $KMnO_4$/Aceton/$H_2O$ ($Z^1$ = CHO) bzw. $H_2SO_4/H_2O$ ($Z^1$ = CN)

c) 1. $SOCl_2$ 2. $AlCl_3/CH_2Cl_2$ d) $Et_3SiH/F_3CCOOH$

Analog lassen sich erfindungsgemäße 3-Fluorfluorene, 2,3-Difluorfluorene, 3,5-Difluorfluorene und 2,3,5-Trifluorfluorene herstellen (Schema 2). Die hierzu benötigten 2-Brombenzaldehyde sind entweder bekannt. (2-Brom-4-fluorbenzaldehyd: CAS 59142-68-6) oder man erhält sie durch Reduktion der entsprechenden 2-Brombenzoesäuren bzw. Benzonitrile (2-Brom-4,5-difluorbenzoesäure: CAS 64695-84-7; 2-Brom-4,5-difluorbenzonitril: CAS 64695-82-5). Alternativ können die 2-Brombenzoesäuren bzw. 2-Brombenzonitrile wie in Schema 2 aufgeführt auch direkt mit einer gegebenenfalls in 2-Position fluorsubstituierten Phenylboronsäure zum entsprechenden Biphenylderivat gekoppelt werden (nach US 5,686,608 bzw. Synlett 2000, 6, 829). Durch Verseifung der Biphenyl-2-carbonitrile erhält man die benötigten Biphenyl-2-carbonsäuren.

Schema 3

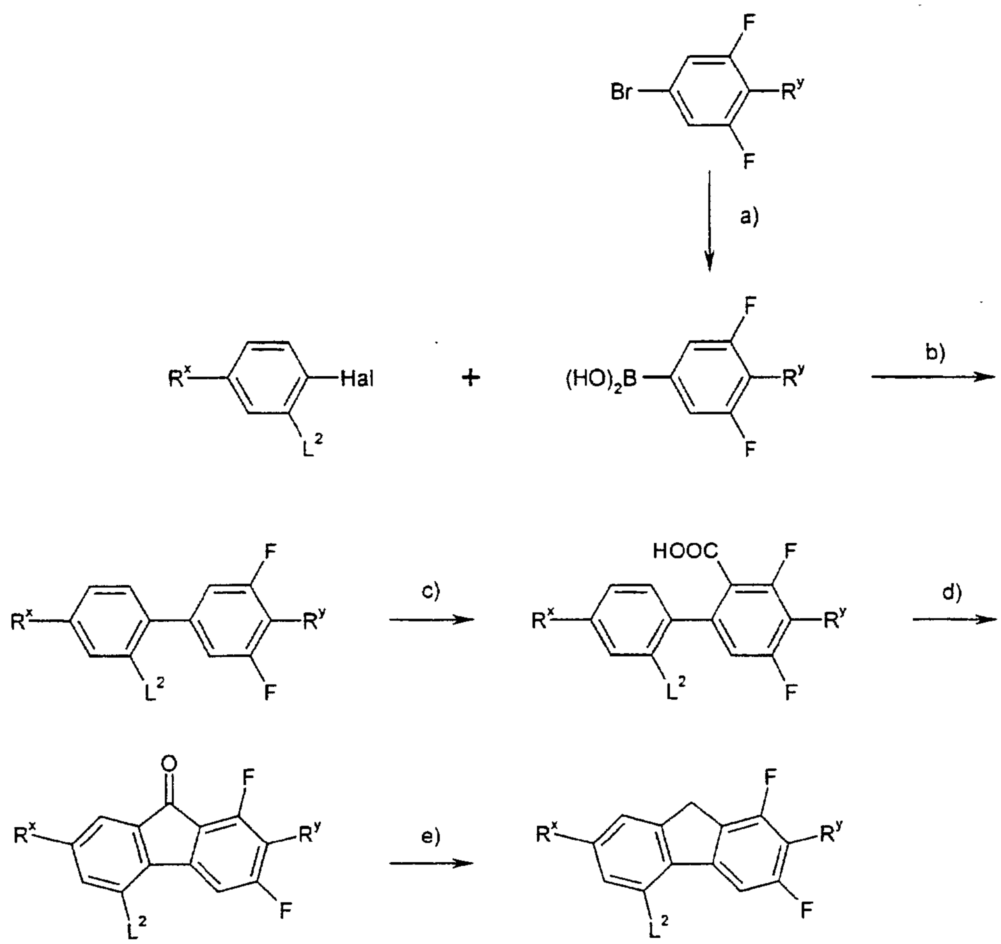


a) 1. Mg/THF 2. $B(OMe)_3$ 3. $H_3O^+$ b) $Pd(PPh_3)_4/Na_2CO_3$/Toluol/EtOH/$H_2O$

c) *n*-BuLi/THF/Hexan 2. $CO_2$ 3. $H_3O^+$ d) 1. $SOCl_2$ 2. $AlCl_3/CH_2Cl_2$ e) $Et_3SiH$/ $F_3CCOOH$

Erfindungsgemäße 1,2,3-Trifluorfluorene, 1,2,3,5-Tetrafluorfluorene, 1,3-Difluorfluorene und 1,3,5-Trifluorfluorene lassen sich beispielsweise nach Schema 3 ausgehend von käuflichem 1-Brom-3,4,5-trifluorbenzol, 4-Brom-2,6-difluoranisol oder in der Literatur (DE 4221152 C1) beschriebenem 4-Brom-2,6-difluor-1-(trifluormethoxy)benzol (CAS: 115467-07-7) herstellen.

In den Schemata 1 bis 3 bedeutet die Gruppe $R^x$ die Gruppierung $R^1(-A^1-M^1)_m$ oder eine geeignete, gegebenenfalls geschützte Vorstufe hiervon (beispielsweise Br oder $OSO_2CF_3$ im Fall Hal = I; Benzyloxy; Tetrahydopyranyloxy; *tert*-Butyldiphenylsilanyloxy), die in späteren Schritten nach an sich bekannten, dem Fachmann geläufigen Methoden in diese Gruppe überführt werden kann. Entsprechend bedeutet die Gruppe $R^y$ die Gruppierung $X^1$ oder eine geeignete, gegebenenfalls geschützte Vorstufe hiervon. Die Gruppe Hal bedeutet Cl, Br, I oder $OSO_2CF_3$.

Beispielsweise können nach Schema 1, 2 und 3 erhaltene 7-methoxy- und 7-bromsubstituierte Fluorene ($R^x$ = Br oder $H_3CO$) gemäß Schema 4 und 5 zu den erfindungsgemäßen Verbindungen der Formel (I) umgesetzt werden.

Schema 4

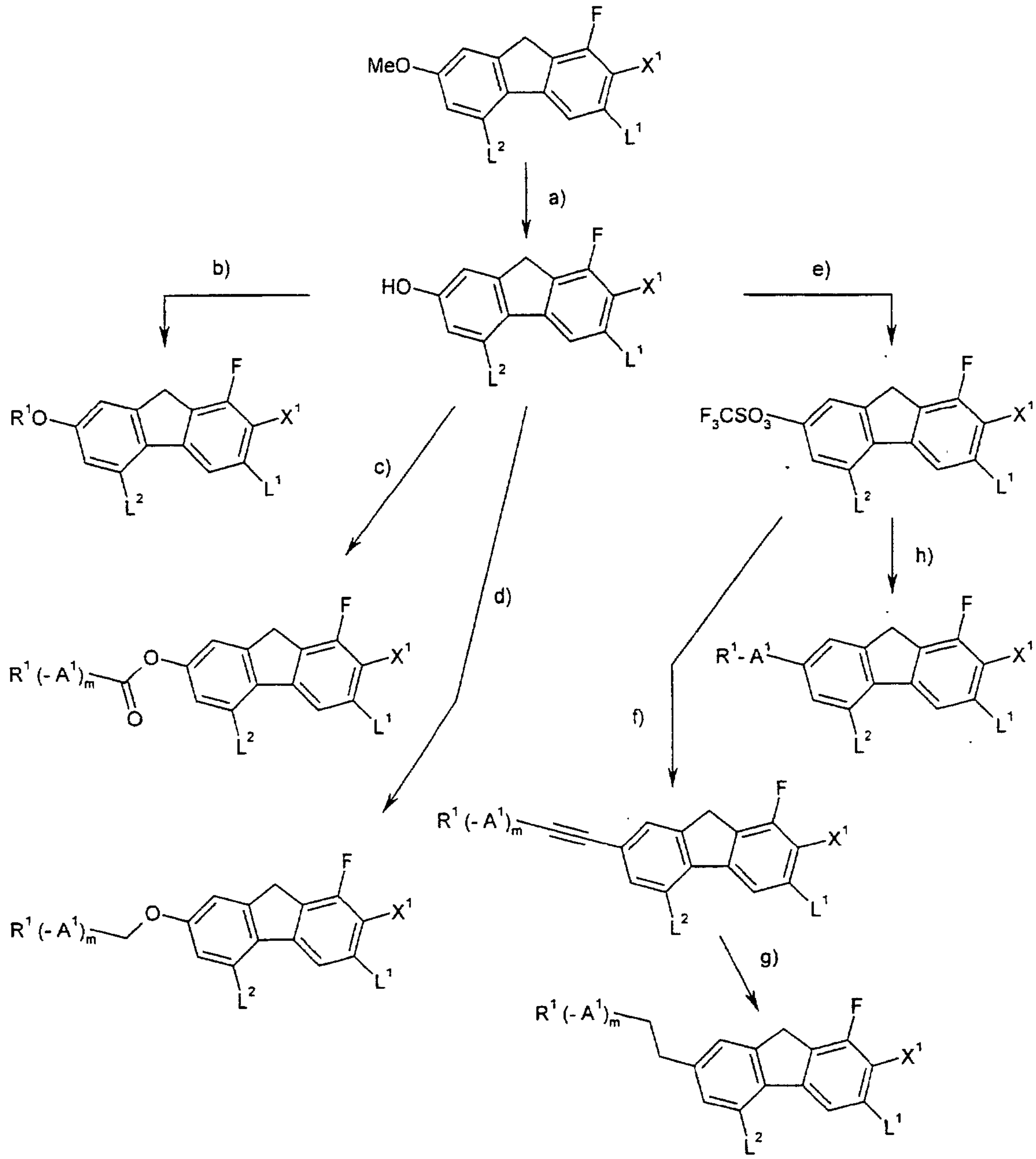


a) HBr/$CH_3COOH$ b) $R^1Br$/$K_2CO_3$/MEK c) $R^1(-A^1)_m$-COOH/DCC/DMAP/ $CH_2Cl_2$

d) $R^1(-A^1)_m$-$CH_2OH$/DEAD/$PPh_3$/THF e) $(F_3CSO_2)_2O$ / Pyridin

f) $R^1(-A^1)_m$-C≡CH/$Pd(PPh_3)_2Cl_2$/ CuI/$NEt_3$ g) $H_2$/Pd-C/THF

h) $R^1$-$A^1$-$B(OH)_2$/$Pd(PPh_3)_4$/$Na_2CO_3$/Toluol/EtOH/$H_2O$

Schema 5

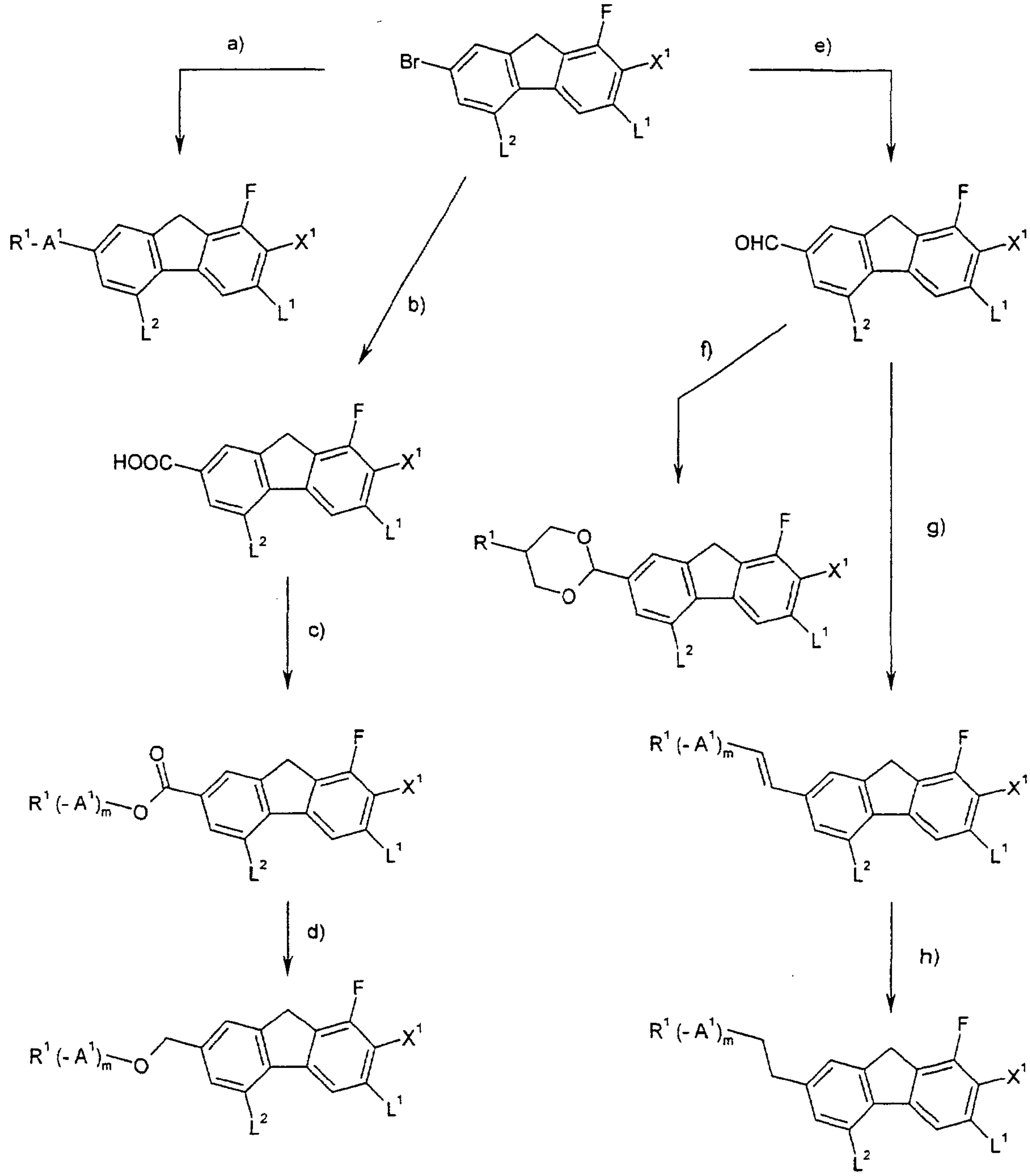


a) $R^1$-$A^1$-$B(OH)_2$/$Pd(PPh_3)_4$/$Na_2CO_3$/Toluol/EtOH/$H_2O$

b) 1. Mg/THF 2. $CO_2$ 3. $H_3O^+$ c) $R^1(-A^1)_m$-OH/DCC/DMAP/$CH_2Cl_2$

d) $NaBH_4$/$BF_3$/THF/Diglyme e) Mg/THF 2. DMF 3. $H_3O^+$

f) $R^1$-$CH(CH_2OH)_2$/4-TsOH/Toluol

g) $R^1(-A^1)_m$-$CH_2$-$P(Ph_3)Br$/KO*t*Bu/THF h) $H_2$/Pd-C/THF

In Position 7 durch $R^4$ substituierte 1-Fluorfluorene und 1,3-Difluorfluorene können durch Metallierung der

Position 2 und nachfolgende Umsetzung mit Elektrophilen (z. B. Trimethylborat, DMF, $CO_2$, *n*-Alkylaldehyden, 4-*n*-Alkylcyclohexanonen, Iod, Brom) zu Vorstufen umgesetzt werden, aus denen sich die erfindungsgemäßen Verbindungen der Formel (I) herstellen lassen (Schemata 6 bis 9).

Schema 6

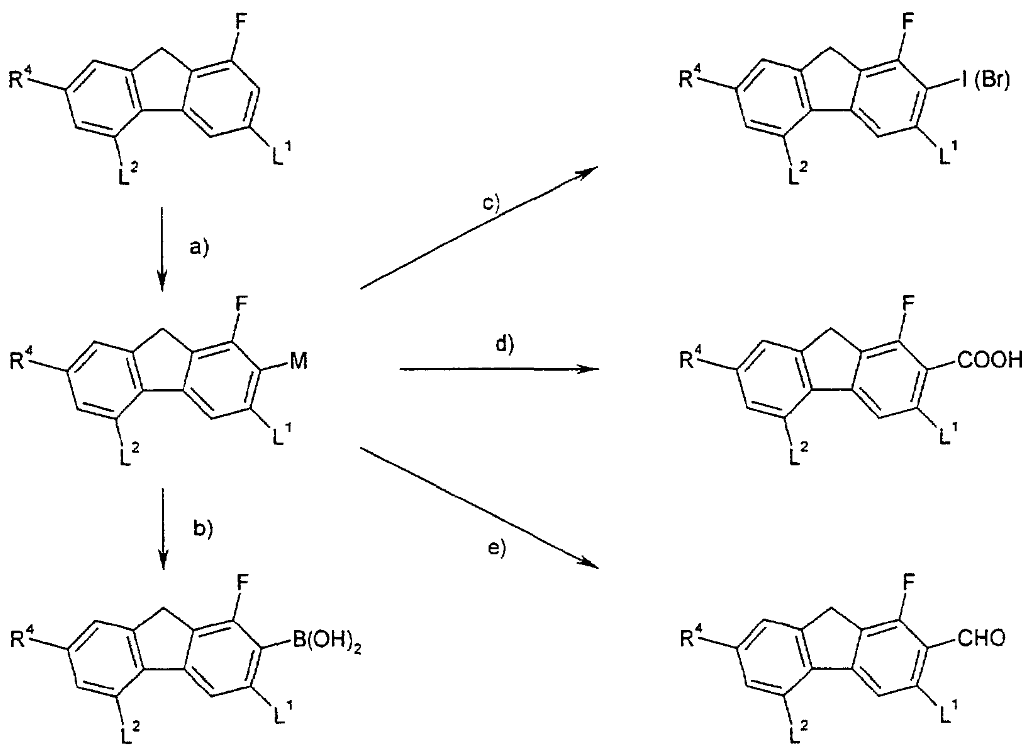


M = Li, K a) *n*-BuLi/THF/Hexan oder *n*-BuLi/KO*t*Bu/THF/Hexan oder *sec*-BuLi/THF/Cyclohexan oder LiTMP/THF/Hexan b) 1. $B(OMe)_3$ 2. $H_3O^+$ c) $I_2$ (bzw. $Br_2$) d) 1. $CO_2$ 2. $H_3O^+$ e) 1. DMF 2. $H_3O^+$

Schema 7

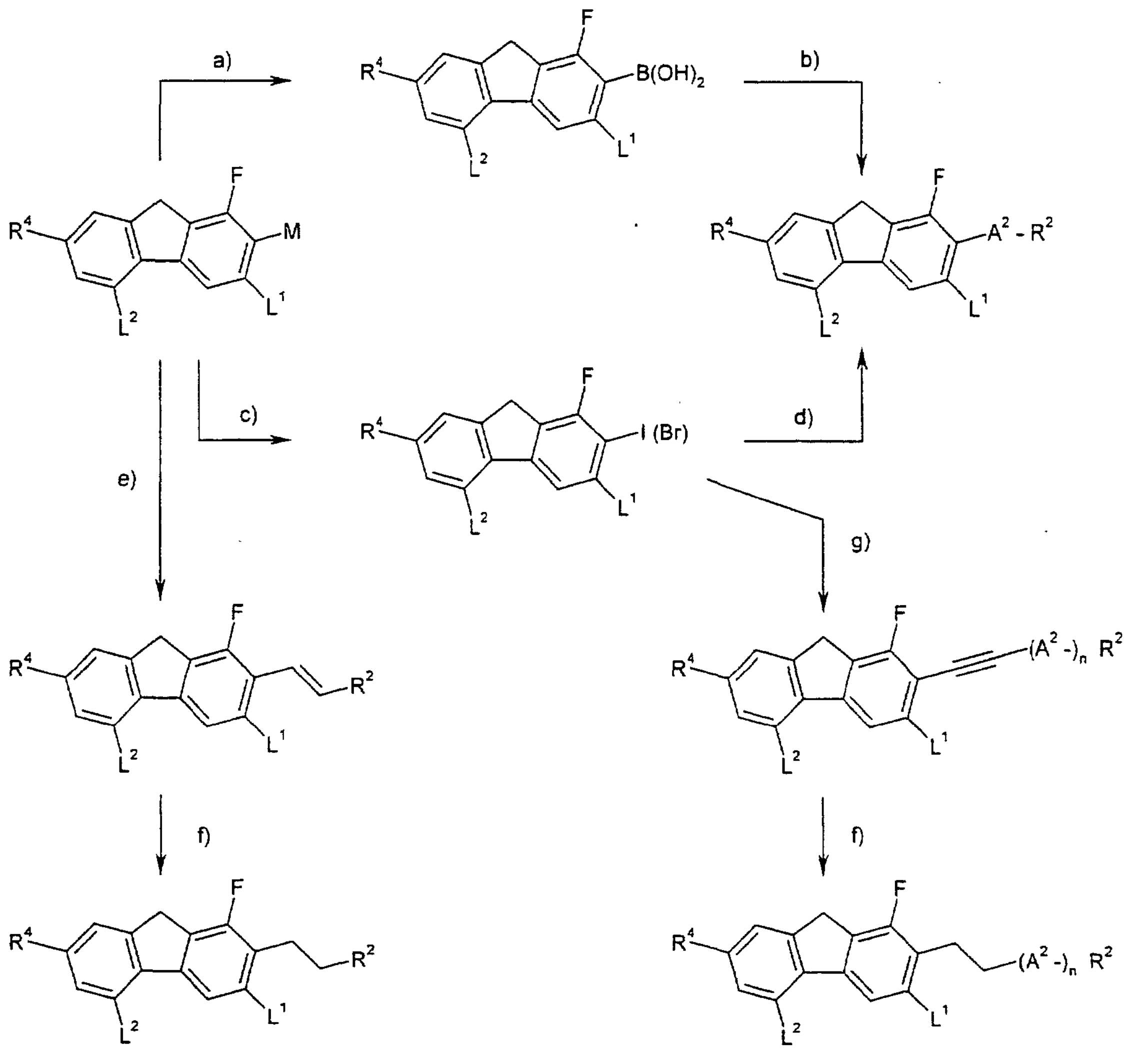


a) 1. $B(OMe)_3$ 2. $H_3O^+$ b) $R^2$-$A^2$-Hal/$Pd(PPh_3)_4$/$Na_2CO_3$/Toluol/EtOH/$H_2O$

c) $I_2$ (oder $Br_2$) d) $R^2$-$A^2$-$B(OH)_2$/$Pd(PPh_3)_4$/$Na_2CO_3$/Toluol/EtOH/$H_2O$

e) 1. $R^2$-$CH_2CHO$ 2. $H_3O^+$ 3. 4-TsOH/Toluol f) $H_2$/Pd-C/THF

g) $R^2$-$(A^2)_n$-C≡CH/$Pd(PPh_3)_2Cl_2$/CuI/$NEt_3$

Schema 8

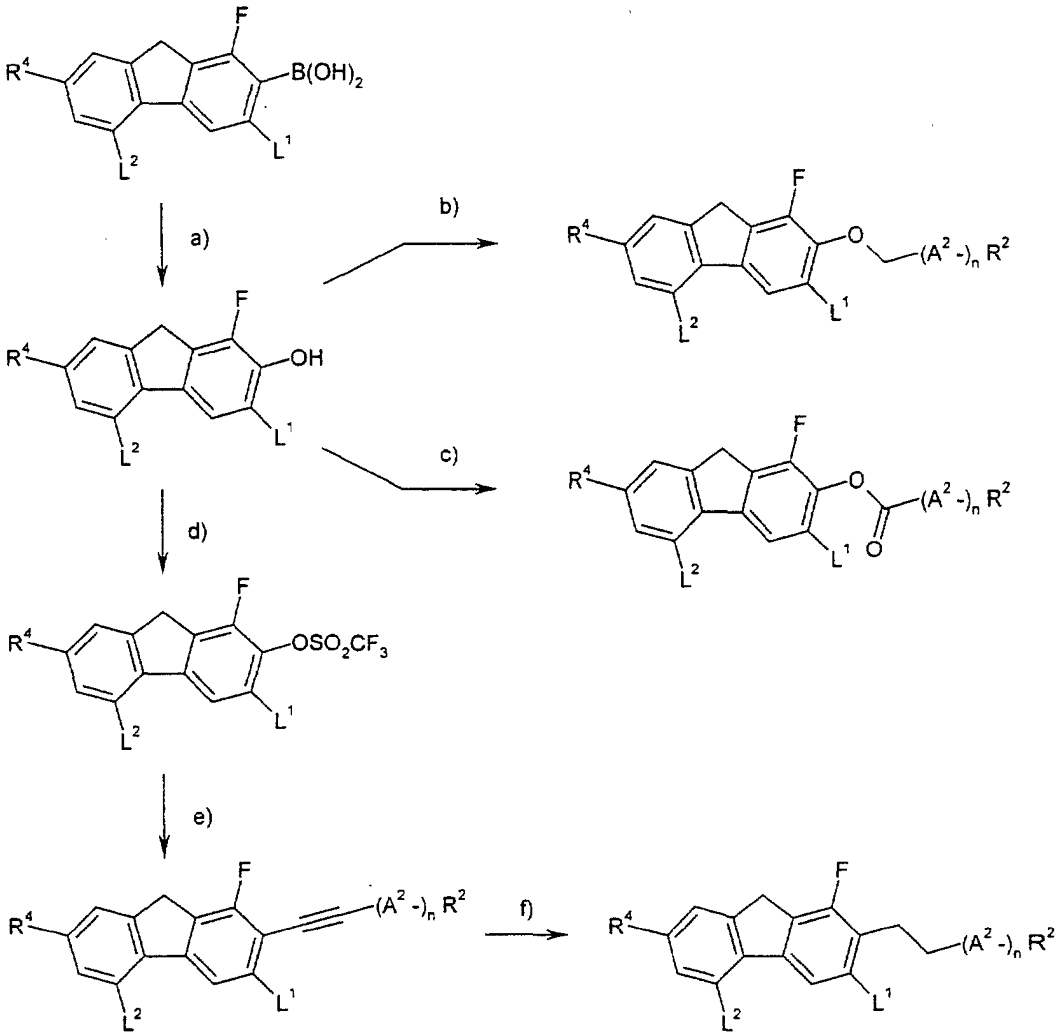


a) $H_2O_2$/MTBE b) $R^2(-A^2)_n-CH_2OH$/DEAD/$PPh_3$/THF oder $R^2(-A^2)_n-CH_2Br/K_2CO_3$/MEK c) $R^2(-A^2)_n$-COOH/DCC/DMAP/ $CH_2Cl_2$ d) $(F_3CSO_2)_2O$/Pyridin e) $R^2(-A^2)_n-C\equiv CH/Pd(PPh_3)_2Cl_2$/CuI/$NEt_3$ f) $H_2$/Pd-C/THF

Schema 9

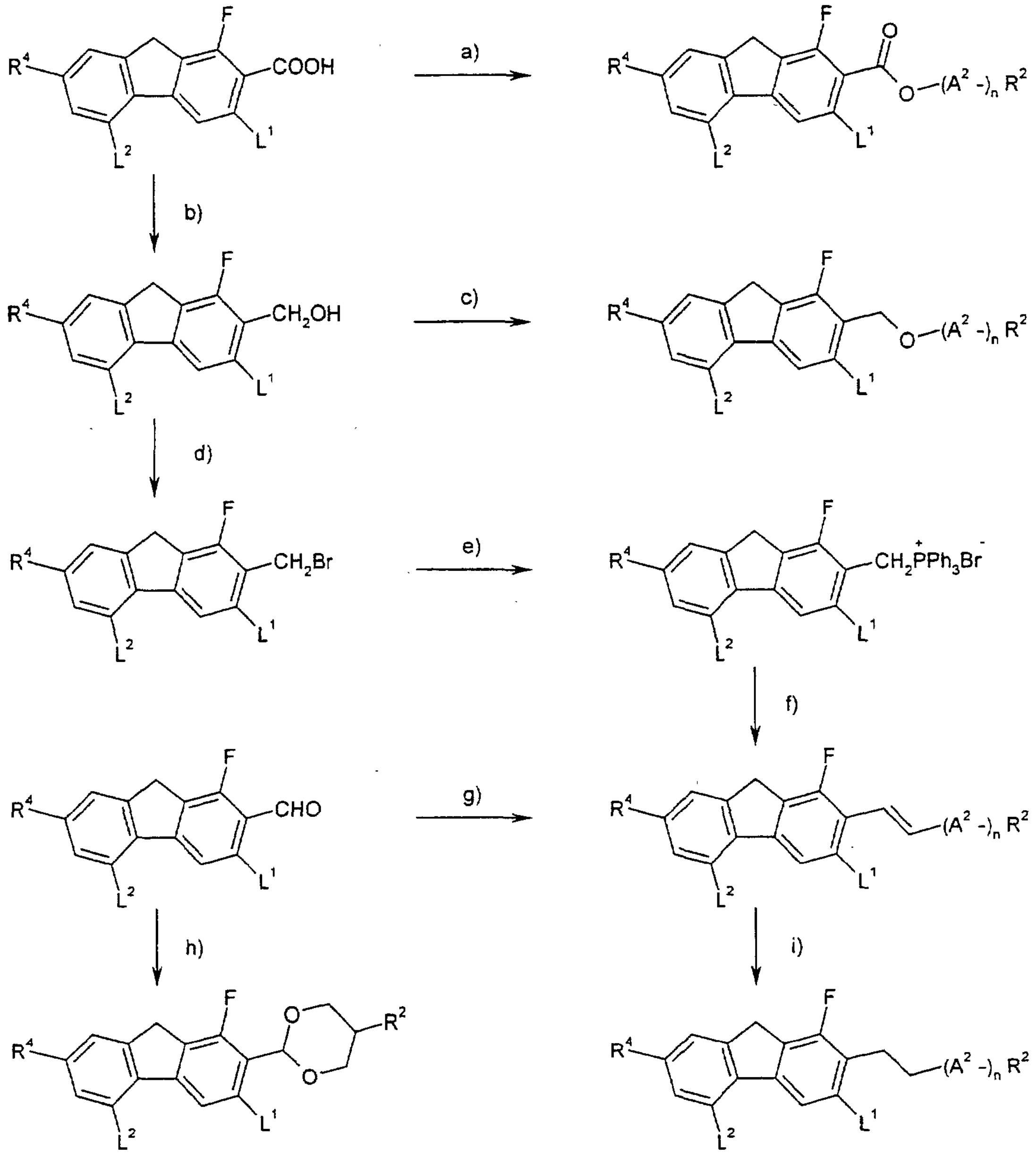


a) $R^2(\text{-}A^2)_n$-OH/DCC/DMAP/$CH_2Cl_2$ b) $LiAlH_4$/THF 2. $H_3O^+$

c) $R^2(\text{-}A^2)_n$-OH/DEAD/$PPh_3$/THF d) $PBr_3$/$CHCl_3$ e) $PPh_3$/Xylol

f) $R^2(\text{-}A^2)_n$-CHO/KO*t*Bu/THF g) $R^2(\text{-}A^2)_n$-$CH_2P(Ph_3)Br$/KO*t*Bu/THF

h) $R^2$-$CH(CH_2OH)_2$/4-TsOH/Toluol i) $H_2$/Pd-C/THF

Die Herstellung der für die Synthesen nach Schema 1 bis 9 benötigten, beispielsweise alkyl-, alkenyl- oder alkoxy-substituierten, ggf. zusätzlich fluorierten Benzoesäuren, Cyclohexancarbonsäuren und Phenylacetylene (Schemata 4, 7 und 8), Phenylboronsäuren, Pyridylboronsäuren (Schemata 1 bis 5 und 7), Brombenzole, Brompyrimidine (Schema 7) und 2-Alkylpropan-1,3-diole (Schema 5, 9) und deren Umsetzungen sind dem Fachmann bekannt und beispielsweise in WO 96/00710, WO 96/30344, Liq. Cryst. 1995, 18, 1, Mol. Cryst. Liq. Cryst. 1991, 204, 43, Liq. Cryst. 1997, 23, 389, Synthesis 1996, 589, WO 92/11241, EP-A-0 665 825 und J. Mater. Chem. 1999, 9, 1669 beschrieben. Entsprechend substituierte Benzylalkohole und (Hydroxymethyl)-cyclohexane $R^2$-$A^2$-$CH_2OH$ lassen sich beispielswei-

se aus den entsprechenden Benzoesäuren bzw. Cyclohexancarbonsäuren $R^2$-$A^2$-COOH durch Reduktion mit Lithiumaluminiumhydrid (allg. Arbeitsvorschrift: Organikum, VEB Deutscher Verlag der Wissenschaften, 15. Aufl., Berlin 1984, Kap. 7.3.4) erhalten. Deren Bromierung mit Phosphortribromid (analog J. Org. Chem. 1984, 49, 2534-2540) liefert die in Schema 5 benötigten Benzylbromide bzw. (Brommethyl)-cyclohexane $R^2$-$A^2$-$CH_2Br$. Anschließende Umsetzung mit Triphenylphosphin in Xylol liefert die in Schema 4 erwähnten Triphenylphosphoniumbromide $R^2$-$A^2$-$CH_2$-$P(Ph_3)Br$. Entsprechend substituierte Benzaldehyde und Cyclohexancarboxaldehyde $R^2$-$A^2$-CHO sind beispielsweise durch Reduktion des jeweiligen Carbonsäureesters $R^2$-$A^2$-COOR (Bull. Korean. Chem. Soc. 1999, 20, 1373) oder Oxidation der zuvor genannten Benzylalkohole und (Hydroxymethyl)-cyclohexane $R^2$-$A^2$-$CH_2OH$ (Tetrahedron Lett. 1968, 30, 3363) erhältlich.

Die Synthese spezieller Reste $X^1$ bzw. $X^2$ erfolgt beispielsweise nach DE-A-195 28 085, DE-A-195 32 292 und DE-A-196 54 487. Erfindungsgemäße Verbindungen der Formel (I) mit 1-Cyclohexen-1,4-diyl oder 2-Fluor-1-cyclohexen-1,4-diyl- oder 4-Fluor-3-cyclohexen-1-yl-Einheit werden wie in Liq. Cryst. 1997, 23, 69, DE-A-44 27 266, DE-A-196 07 996, DE-A-195 28 665 und EP-A-0 736 513 beschrieben hergestellt. Was die Synthese spezieller Reste $R^1$ und $R^2$ angeht, sei zusätzlich beispielsweise verwiesen auf EP-A-0 355 008 (für Verbindungen mit siliciumhaltigen Seitenketten), US 4,798,680 (für optisch aktive Verbindungen mit 2-Fluoralkyloxy-Einheit), EP-A-0 552 658 (für Verbindungen mit Cyclohexylpropionsäureresten) und EP-A-0 318 423 (für Verbindungen mit Cyclopropylgruppen in der Seitenkette).

Die Erfindung wird durch die nachstehenden Beispiele näher erläutert.

Beispiel 1:

1,2-Difluor-7-(*trans*-4-*n*-propylcyclohexyl)fluoren

145 mmol 1-Brom-3,4-difluorbenzol werden bei -75°C zu einer Lösung von 152 mmol LiTMP (aus 152 mmol 2,2,6,6-Tetramethylpiperidin und 152 mmol *n*-Butyllithium, 1,6 M Lösung in Hexan) in 280 ml THF getropft. Man beläßt 4 h bei dieser Temperatur und versetzt dann tropfenweise mit 174 mmol DMF. Anschließend läßt man die Reaktionsmischung auftauen, hydrolysiert mit Wasser, säuert mit Salzsäure an und extrahiert mit *tert*-Butylmethylether. Die vereinigten organischen Extrakte werden mit gesättigter Natriumchloridlösung gewaschen und mit Natriumsulfat getrocknet. Nach dem Entfernen der Lösemittel im Vakuum wird das Rohprodukt durch Chromatografie an Kieselgel (Eluent: Dichlormethan/ Heptan 1: 1) und Umkristallisation aus Heptan gereinigt. Man erhält 2-Brom-5,6-difluorbenzaldehyd in Form leicht gelblicher Kristalle. - Schmp.: 40-43,5°C.

Eine Mischung aus 127 mmol 2-Brom-5,6-difluorbenzaldehyd, 146 mmol 4-(*trans*-4-*n*-Propylcyclohexyl)phenylboronsäure, 298 mmol Cäsiumfluorid (wasserfrei) und 4,4 mmol Tetrakis(triphenylphosphin)palladium(0) in 630 ml Dimethoxyethan wird zum Sieden erhitzt, bis die Reaktion vollständig ist. Nach dem Abkühlen wird die Reaktionsmischung mit *tert*-Butylmethylether und Wasser versetzt. Die organische Phase wird mit Wasser und gesättigter NaCl-Lösung gewaschen, mit Natriumsulfat getrocknet, und die Lösemittel werden im Vakuum entfernt. Nach säulenchromatografischer Reinigung des Rohproduktes (Kieselgel, Heptan/Ethylacetat 9:1), gefolgt von einer Umkristallisation aus Heptan wird 3,4-Difluor-4'-(*trans*-4-*n*-propylcyclohexyl)biphenyl-2-carbaldehyd in Form farbloser Kristalle erhalten.

Eine Lösung von 7,6 g (22 mmol) des Aldehyds in 150 ml Aceton wird zum Sieden erhitzt und eine Lösung von 4,6 g (29 mmol) Kaliumpermanganat in 100 ml Wasser wird langsam zugetropft. Anschließend wird noch 1 h unter Rückfluß nachgerührt. Nach dem Abkühlen versetzt man die braune Reaktionsmischung mit Natriumsulfitlösung und verdünnter Schwefelsäure, bis die Lösung klar wird. Der größte Teil des Acetons wird am Rotationsverdampfer entfernt und der Rückstand wird mit tert-Butylmethylether extrahiert.

Die vereinigten organischen Phasen werden mit gesättigter Natriumchloridlösung gewaschen, mit Natriumsulfat getrocknet, mit Aktivkohle entfärbt, und das Lösemittel wird am Rotationsverdampfer entfernt. Nach Umkristallisation aus Heptan erhält man 3,4-Difluor-4'-(*trans*-4-*n*-propylcyclohexyl)biphenyl-2-carbonsäure in Form farbloser Kristalle.

Man legt 5,3 g (15 mmol) 3,4-Difluor-4'-(*trans*-4-*n*-propylcyclohexyl)biphenyl-2-carbonsäure vor, gibt 10 ml Thionylchlorid zu und rührt unter Erwärmen, bis keine Gasentwicklung mehr zu beobachten ist. Nach dem Abkühlen wird überschüssiges Thionylchlorid im Vakuum abdestilliert. Eine Lösung des erhaltenen Säurechlorids in 50 ml Dichlormethan wird unter Rühren und Kühlen langsam innerhalb 1 h bei 0 bis 5 °C zu einer Suspension von 2,4 g (18 mmol) Aluminiumchlorid in 200 ml Dichlormethan getropft. Anschließend wird noch 1 h bei Raumtemperatur nachgerührt. Man gibt die Reaktionsmischung auf Eis und bringt ausgeschiedenes Aluminiumhydroxid mit konzentrierte Salzsäure in Lösung. Die organische Phase wird mit Wasser und verdünnter Natronlauge gewaschen, mit Natriumsulfat getrocknet, und das Lösemittel wird im Vakuum entfernt. Das Rohprodukt reinigt man säulenchromatographisch (Kieselgel, Dichlormethan) und durch Umkristallisation. Man erhält 1,2-Difluor-7-(*trans*-4-*n*-propylcyclohexyl)-9-fluorenon in Form leicht gelblicher Kristalle.

3,1 g (9 mmol) 1,2-Difluor-7-(*trans*-4-*n*-propylcyclohexyl)-fluoren-9-on werden in 20 ml Trifluoressigsäure gelöst und 3,2 g (27 mmol) Triethylsilan langsam bei Raumtemperatur zugetropft. Man rührt anschließend 6 h unter Rückfluß. Nach dem Abkühlen wird das Reaktionsgemisch auf Eis/Wasser gegeben. Man trennt die Phasen, extrahiert die wässrige Phase mit *tert*-Butylmethylether, wäscht die vereinigten organischen Phasen mit Wasser und verdünnter Natriumhydrogencarbonatlösung und trocknet mit Natriumsulfat. Das Lösemittel wird im Vakuum entfernt und das Rohprodukt säulenchromatographisch an Kieselgel (*n*-Heptan) gereinigt. Nach Umkristallisation aus *n*-Heptan erhält man 1,2-Difluor-7-(*trans*-4-*n*-propylcyclohexyl)fluoren in Form farbloser Kristalle.

Durch Einsatz von 4-(*trans*-4-*n*-Pentylcyclohexyl)phenylboronsäure, 4-*n*-Pentylphenylboronsäure, 4-*n*-Propylphenylboronsäure, 4-*n*-Butyloxyphenylboronsäure, 4-*n*-Octyloxyphenylboronsäure, 4'-*n*-Pentylbiphenyl-4-boronsäure, 4'-*n*-Propylbiphenyl-4-boronsäure, 4'-*n*-Hexyloxybiphenyl-4-boronsäure (jeweils erhalten durch Reaktion der entsprechenden Bromverbindungen mit Magnesium in THF, Umsetzung mit Trimethylborat und saurer Hydrolyse) an Stelle von 4-(*trans*-4-*n*-Propylcyclohexyl)phenylboronsäure in der zweiten Stufe können analog erhalten werden:

Beispiel 2: 1,2-Difluor-7-(*trans*-4-*n*-pentylcyclohexyl)fluoren

Beispiel 3: 1,2-Difluor-7-*n*-pentylfluoren

Beispiel 4: 1,2-Difluor-7-*n*-propylfluoren

Beispiel 5: 7-*n*-Butyloxy-1,2-difluorfluoren

Beispiel 6: 1,2-Difluor-7-*n*-octyloxyfluoren

Beispiel 7: 1,2-Difluor-7-(4-*n*-pentylphenyl)fluoren

Beispiel 8: 1,2-Difluor-7-(4-*n*-propylphenyl)fluoren

Beispiel 9: 1,2-Difluor-7-(4-*n*-hexyloxyphenyl)fluoren

Beispiel 10:

1,2,3-Trifluor-7-*n*-pentylfluoren

Eine Mischung aus 25 g (130 mmol) 4-*n*-Pentylphenylboronsäure, 25 g (118 mmol) 1-Brom-3,4,5-trifluorbenzol, 24,9 g (237 mmol) Natriumcarbonat und 1,4 g (1mol%) Tetrakis(triphenylphosphin)palladium(0) wird zum Sieden erhitzt, bis die Reaktion vollständig ist (5 h). Nach dem Abkühlen versetzt man mit 100 ml Wasser, trennt die Phasen und extrahiert die wässrige Phase mit Toluol. Die vereinigten organischen Phasen werden mit gesättigter NaCl-Lösung gewaschen, mit Natriumsulfat getrocknet, und die Lösemittel werden im Vakuum entfernt. Das Rohprodukt wird an Kieselgel (Dichlormethan/Heptan 2:3) chromatografiert. Man erhält 3,4,5-Trifluor-4'-*n*-pentylbiphenyl als farbloses Öl.

Eine Lösung von 8,3 g (30 mmol) 3,4,5-Trifluor-4'-*n*-pentylbiphenyl in 100 ml Tetrahydrofuran wird unter Schutzgasatmosphäre auf ≤ -70°C gekühlt, und 21 ml (33 mmol) *n*-Butyllithium (1,6 M Lösung in Hexan) werden so zugetropft, daß die Innentemperatur stets unterhalb -70°C liegt. Anschließend rührt man noch 3 h bei dieser Temperatur nach. Nun leitet man Kohlendioxid bei ≤ -70°C ein und läßt anschließend langsam auftauen. Die Reaktionsmischung wird mit 100 ml Wasser versetzt und mit konzentrierte Salzsäure auf pH 1 angesäuert. Man extrahiert mit *tert*-Butylmethylether, wäscht die vereinigten organischen Phasen mit gesättigter Natriumchloridlösung, trocknet mit Natriumsulfat und entfernt die Lösemittel im Vakuum. Nach Chromatografie an Kieselgel mit Dichlormethan als Eluent erhält man 3,4,5-Trifluor-4'-*n*-pentylbiphenyl-2-carbonsäure.

Die Umsetzung der 3,4,5-Trifluor-4'-*n*-pentylbiphenyl-2-carbonsäure (Friedel-Crafts-Cyclisierung und Reduktion mit Triethylsilan) zu 1,2,3-Trifluor-7-*n*-pentylfluoren erfolgt analog zu Beispiel 1.

Durch Einsatz von 4-(*trans*-4-*n*-Propylcyclohexyl)phenylboronsäure, *4-(trans*-4-*n*-Pentylcyclohexyl)phenylboronsäure, 4-*n*-Propylphenylboronsäure, 2-Fluor-4'-*n*-pentylbiphenyl-4-boronsäure, 2-Fluor-4-(*trans*-4-*n*-propylcyclohexyl)phenylboronsäure, 4-Methoxyphenylboronsäure und 4-Methoxy-2-fluorphenylboronsäure an Stelle von 4-*n*-Pentylphenylboronsäure können analog erhalten werden:

Beispiel 11: 1,2,3-Trifluor-7-(*trans*-4-*n*-propylcyclohexyl)fluoren

Beispiel 12: 1,2,3-Trifluor-7-(*trans*-4-*n*-pentylcyclohexyl)fluoren

Beispiel 13: 1,2,3-Trifluor-7-*n*-propylfluoren

Beispiel 14: 1,2,3,5-Tetrafluor-7-(4-*n*-pentylphenyl)-fluoren

Beispiel 15: 1,2,3,5-Tetrafluor-7-(*trans*-4-*n*-propylcyclohexyl)fluoren

Beispiel 16: 1,2,3-Trifluor-7-methoxyfluoren

Beispiel 17: 1,2,3,5-Tetrafluor-7-methoxyfluoren

Beispiel 18:

7-Allyloxy-1,2,3-trifluorfluoren

Eine Mischung aus 43 mmol 1,2,3-Trifluor-7-methoxyfluoren (Beispiel 16) in 90 ml Eisessig und 77 ml 48 prozentiger Bromwasserstoffsäure wird 4,5 h zum Sieden erhitzt. Anschließend wird auf 1 l Eis/Wasser gegeben, der Niederschlag abgesaugt und mit Wasser neutral gewaschen und getrocknet. Nach Umkristallisation aus Heptan/Aceton erhält man 6,7,8-Trifluorfluoren-2-ol.

Zu einer Mischung von 18 mmol 6,7,8-Trifluorfluoren-2-ol und 54 mmol wasserfreiem Kaliumcarbonat in 100 ml Aceton gibt man 25 mmol Allylbromid und erhitzt anschließend 5 h unter Rückfluß. Die Mischung wird in 100 ml Wasser gegeben, und der Niederschlag wird abgesaugt, mit Wasser neutral gewaschen und getrocknet. Die Reinigung des Rohproduktes erfolgt durch Chromatografie an Kieselgel (Dichlormethan/Heptan) und Umkristallisation aus Heptan. Man erhält 7-Allyloxy-1,2,3-trifluorfluoren.

Analog können ausgehend von 6,7,8-Trifluorfluoren-2-ol erhalten werden:

Beispiel 19: 1,2,3-Trifluor-7-*n*-hexyloxyfluoren

Beispiel 20: 7-*n*-Butoxy-1,2,3-trifluorfluoren

Analog Beispiel 18 bis 20, jedoch unter Einsatz von 1,2,3,5-Tetrafluor-7-methoxyfluoren (Beispiel 17) an Stelle von 1,2,3-Trifluor-7-methoxyfluoren (Beispiel 16), können erhalten werden:

Beispiel 21: 7-Allyloxy-1,2,3,5-tetrafluorfluoren

Beispiel 22: 7-*n*-Butoxy-1,2,3,5-tetrafluorfluoren

Beispiel 23:

1,2,3-Trifluor-7-(4-*n*-propylphenyl)-fluoren

Eine Lösung von 22 mmol 6,7,8-Trifluorfluoren-2-ol (aus Beispiel 18) in 80 ml Pyridin werden bei 0 bis 10°C mit 28 mmol Trifluormethansulfonsäureanhydrid versetzt und 4 h bei Raumtemperatur nachgerührt. Anschließend gibt man die Reaktionsmischung auf Eis/Wasser, saugt den Niederschlag ab und wäscht mit verdünnter Salzsäure und Wasser nach. Das Rohprodukt wird durch Umfällen in Aceton/Wasser und Umkristallisation in Heptan gereinigt. Man erhält Trifluormethansulfonsäure-(6,7,8-trifluorfluoren-2-yl)-ester.

19 mmol 4-*n*-Propylphenylboronsäure, 17 mmol Trifluormethansulfonsäure-(6,7,8-trifluorfluoren-2-yl)-ester, 34 mmol Natriumcarbonat und 0,2 mmol Tetrakis(triphenylphosphin)palladium(0) werden analog zur für Beispiel 10 beschriebenen Vorgehensweise umgesetzt und gereinigt. Man erhält 1,2,3-Trifluor-7-(4-*n*-propylphenyl)-fluoren.

Durch Einsatz von 2-Fluor-4-*n*-pentyloxyphenylboronsäure und 2-Fluor-4-*n*-propylphenylboronsäure an Stelle von 4-*n*-Propylphenylboronsäure können analog erhalten werden:

Beispiel 24: 1,2,3-Trifluor-7-(2-fluor-4-*n*-pentyloxyphenyl)-fluoren

Beispiel 25: 1,2,3-Trifluor-7-(2-fluor-4-*n*-propylphenyl)-fluoren

Analog zu Beispiel 10 bis 15, jedoch ausgehend von 4-Brom-2,6-difluor-1-(trifluormethoxy)benzol (CAS: 115467-07-7) an Stelle von 1-Brom-3,4,5-trifluorbenzol, können erhalten werden:

Beispiel 26: 1,3-Difluor-2-(trifluormethoxy)-7-(4-pentylphenyl)-fluoren

Beispiel 27: 1,3-Difluor-2-(trifluormethoxy)-7-(*trans*-4-*n*-propylcyclohexyl)fluoren

Analog zu Beispiel 1 bis 9, jedoch ausgehend von 2-Brom-4,6-difluorbenzaldehyd (CAS: 154650-59-6) an Stelle von dem in Beispiel 1 nach der ersten Stufe erhaltenen 2-Brom-3,4-difluorbenzaldehyd, können erhalten werden:

Beispiel 28: 1,3-Difluor-7-(4-*n*-pentylphenyl)-fluoren

Beispiel 29: 1,3-Difluor-7-(*trans*-4-*n*-propylcyclohexyl)fluoren

Analog zu Beispiel 1 bis 9, jedoch ausgehend von 2-Brom-4-fluorbenzaldehyd (CAS: 59142-68-6) an Stelle von dem in Beispiel 1 nach der ersten Stufe erhaltenen 2-Brom-3,4-difluorbenzaldehyd, können erhalten werden:

Beispiel 30: 3-Fluor-7-(4-*n*-pentylphenyl)-fluoren

Beispiel 31: 3-Fluor-7-(*trans*-4-*n*-propylcyclohexyl)fluoren

Analog zu Beispiel 1 bis 9, jedoch ausgehend von 2-Brom-6-fluorbenzaldehyd (CAS 59142-68-6) an Stelle von dem in Beispiel 1 nach der ersten Stufe erhaltenen 2-Brom-3,4-difluorbenzaldehyd, kann erhalten werden:

Beispiel 32: 1-Fluor-7-(*trans*-4-*n*-propylcyclohexyl)fluoren

Analog zu Beispiel 30 und 31, jedoch ausgehend von 2-Fluor-4-*n*-pentyl-phenylboronsäure und 2-Fluor-4-(*trans*-4-*n*-propylcyclohexyl)phenylboronsäure an Stelle von (*trans*-4-*n*-Propylcyclohexyl)phenylboronsäure, können erhalten werden:

Beispiel 33: 3,5-Difluor-7-(4-*n*-pentylphenyl)-fluoren

Beispiel 34: 3,5-Difluor-7-(*trans*-4-*n*-propylcyclohexyl)fluoren

Beispiel 35:

Eine nematische Testmischung MLC-9000-100 (Hersteller: Merck KGaA, Darmstadt) wird mit 5% der Verbindung aus Beispiel 10 versetzt; man erhält folgende Verbesserungen gegenüber den in Klammern benannten Vergleichswerten der Mischung MLC-9000-100 ($\gamma$ = Rotationsviskosität):
$\Delta n$ = 0.122 (0.1137); $\gamma$ = 189 mPas (201 mPas)

Beispiel 36

Eine chiral-smektisch-C-Mischung bestehend aus

| | |
|---|---|
| 2-(4-Heptyloxyphenyl)-5-nonylpyrimidin | 19,6% |
| 5-Nonyl-2-(4-octyloxyphenyl)pyrimidin | 19,6% |
| 5-Nonyl-2-(4-nonyloxyphenyl)pyrimidin | 19,6% |
| 2-(2,3-Difluor-4-heptyloxyphenyl)-5-nonylpyrimidin | 6,5% |
| 2-(2,3-Difluor-4-octyloxyphenyl)-5-nonylpyrimidin | 6,5% |
| 2-(2,3-Difluor-4-nonyloxyphenyl)-5-nonylpyrimidin | 6,5% |
| 5-Hexyloxy-2-(4-hexyloxyphenyl)pyrimidin | 19,6% |

(fortgesetzt)

| | |
|---|---|
| (*S*)-4-[4'-(2-Fluoroctyloxy)biphenyl-4-yl]-1-heptylcyclohexancarbonitril | 2,0% |

wird mit 5% der Verbindung aus Beispiel 9 versetzt; die resultierende Mischung weist eine 8%ige Verbesserung der Schaltzeit auf.

**Patentansprüche**

**1.** Verbindung der Formel (I)

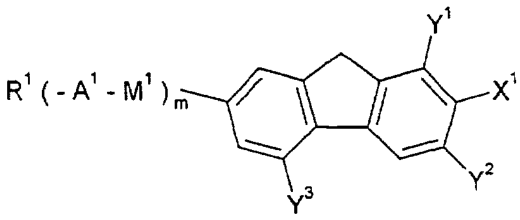

(I)

mit den Bedeutungen:

$Y^1$, $Y^2$, $Y^3$ sind gleich oder verschieden H oder F, mit der Maßgabe, daß mindestens einer von $Y^1$, $Y^2$ gleich F sein muß,

$X^1$ ist gleich F, Cl, CN, $CF_3$, $CHF_2$, $CH_2F$, $OCF_3$, $OCHF_2$, $OCH_2F$, $OCH_2CF_3$, $OCH{=}CF_2$ oder die Gruppierung $(M^2 - A^2 -)_n X^2$,

$X^2$ ist gleich F, Cl, CN, $CF_3$, $CHF_2$, $CH_2F$, $OCF_3$, $OCHF_2$, $OCH_2F$, $OCH_2CF_3$, $OCH{=}CF_2$ oder die Gruppierung $R^2$,

$R^1$, $R^2$ sind gleich oder verschieden
Wasserstoff oder ein geradkettiger oder verzweigter Alkylrest mit 1 bis 16 C-Atomen oder ein geradkettiger oder verzweigter Alkenylrest mit 2 bis 16 C-Atomen, worin

a) eine oder mehrere nicht benachbarte und nicht terminale $CH_2$-Gruppen durch -O-, -C(=O)O-, -O-C(=O)-, -O-C(=O)-O-, -C(=O)- oder $-Si(CH_3)_2-$ ersetzt sein können, und/oder
b) eine $CH_2$-Gruppe durch -C≡C-, Cyclopropan-1,2-diyl oder Cyclobutan-1,3-diyl ersetzt sein kann, und/oder
c) ein oder mehrere H-Atome durch F oder Cl ersetzt sein können, und/oder
d) die terminale $CH_3$-Gruppe durch eine der folgenden chiralen Gruppen (optisch aktiv oder racemisch) ersetzt sein kann:

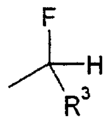

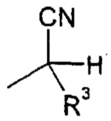

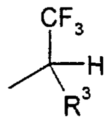

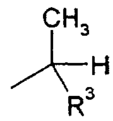

wobei $R^3$ ein geradkettiger oder verzweigter Alkylrest mit 1 bis 12 C Atomen oder ein geradkettiger oder verzweigter Alkenylrest mit 2 bis 12 C-Atomen ist, worin eine oder mehrere nicht benachbarte und nicht terminale $CH_2$-Gruppen durch -O- ersetzt sein können und/oder ein oder mehrere H-Atome durch F oder Cl

ersetzt sein können,

mit der Maßgabe, daß nur einer von $R^1$, $R^2$ gleich Wasserstoff sein kann,

$M^1$, $M^2$ sind gleich oder verschieden
$-C(=O)-O-$, $-O-C(=O)-$, $-CH_2-O-$, $-O-CH_2-$, $-CF_2-O-$, $-O-CF_2-$, $-CH=CH-$, $-CF=CF-$, $-C\equiv C-$, $-CH_2-CH_2-C(=O)-O-$, $-O-C(=O)-CH_2-CH_2-$, $-CH_2-CH_2-$, $-CF_2-CF_2-$, $-(CH_2)_4-$ oder eine Einfachbindung,

$A^1$, $A^2$ sind gleich oder verschieden
1,4-Phenylen, worin ein oder zwei H-Atome durch F, Cl oder CN ersetzt sein können, Pyridin-2,5-diyl, worin ein H-Atom durch F ersetzt sein kann, Pyrimidin-2,5-diyl, 1,4-Cyclohexylen, worin ein oder zwei H-Atome durch $CH_3$ oder F ersetzt sein können, 1-Cyclohexen-1,4-diyl, worin ein H-Atom durch $CH_3$ oder F ersetzt sein kann, oder 1,3-Dioxan-2,5-diyl,

m, n sind unabhängig voneinander 0 oder 1, in Summe jedoch nicht größer als 1.

**2.** Verbindung nach Anspruch 1, **dadurch gekennzeichnet, daß** in der Formel (I) bedeuten:

$X^1$ gleich F, $OCF_3$ oder die Gruppierung $(M^2 - A^2 -)_n X^2$,

$X^2$ gleich F, $OCF_3$ oder die Gruppierung $R^2$,

$R^1$, $R^2$ gleich oder verschieden
ein geradkettiger oder verzweigter Alkylrest mit 1 bis 14 C-Atomen oder ein geradkettiger oder verzweigter Alkenylrest mit 2 bis 14 C-Atomen, worin eine oder zwei nicht benachbarte und nicht terminale $CH_2$-Gruppen durch -O- ersetzt sein können und/oder ein oder mehrere H-Atome durch F ersetzt sein können,

$R^3$ ein geradkettiger oder verzweigter Alkylrest mit 1 bis 10 C-Atomen, worin eine nicht terminale $CH_2$-Gruppe durch -O- ersetzt sein kann,

$M^1$, $M^2$ gleich oder verschieden
$-CH_2-O-$, $-O-CH_2-$, $-CH_2-CH_2-$ oder eine Einfachbindung,

$A^1$, $A^2$ gleich oder verschieden
1,4-Phenylen, worin ein oder zwei H-Atome durch F ersetzt sein können oder 1,4-Cyclohexylen,

m, n unabhängig voneinander 0 oder 1, in Summe jedoch nicht größer als 1.

**3.** Verbindung nach Anspruch 1, ausgewählt aus der Gruppe der Verbindungen der Formeln (I a) bis (I k)

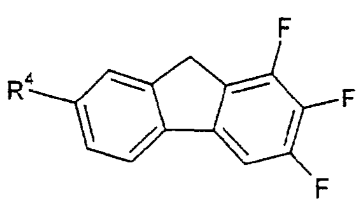

(I a)

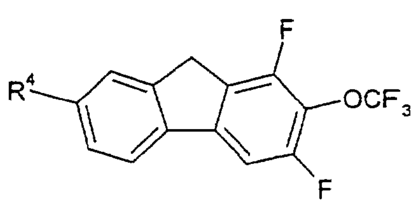

(I b)

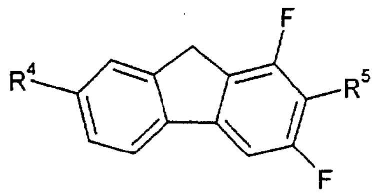

(I c)

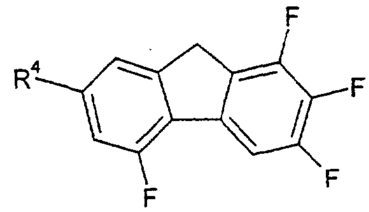

(I d)

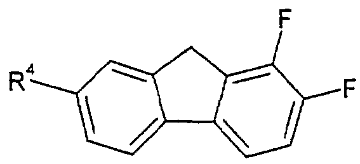

(I e)

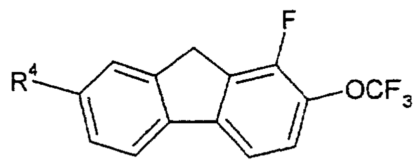

(I f)

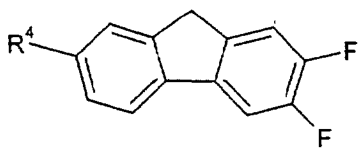

(I g)

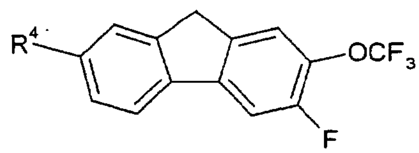

(I h)

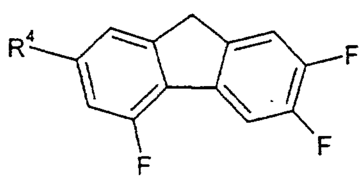

(I i)

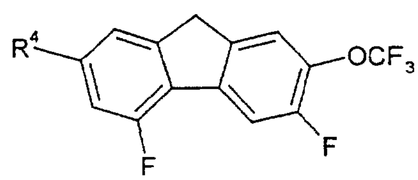

(I k)

mit den Bedeutungen:

$R^4$ ist ein geradkettiger Alkyl-, Alkenyl- oder Alkoxyrest mit 2 bis 12 C-Atomen, worin auch die direkt mit der Fluoreneinheit verknüpfte $CH_2$-Gruppe durch 1,4-Cyclohexylen oder 1,4-Phenylen, welches gegebenenfalls mit ein oder zwei F substituiert ist, ersetzt sein kann,

$R^5$ ist gleich H oder ein geradkettiger Alkyl-, Alkenyl- oder Alkoxyrest mit 2 bis 12 C-Atomen, worin auch die direkt mit der Fluoreneinheit verknüpfte $CH_2$-Gruppe durch 1,4-Cyclohexylen oder 1,4-Phenylen, welches gegebenenfalls mit ein oder zwei F substituiert ist, ersetzt sein kann,

mit der Maßgabe, daß nur in einer von $R^4$, $R^5$ die direkt mit der Fluoreneinheit verknüpfte $CH_2$-Gruppe durch 1,4-Cyclohexylen oder 1,4-Phenylen, welches gegebenenfalls mit ein oder zwei F substituiert ist, ersetzt sein kann.

**4.** Verbindungen nach Anspruch 3, ausgewählt aus der Gruppe der Verbindungen der Formeln (I a) bis (I f), mit den

Bedeutungen:

$R^4$ ist ein geradkettiger Alkyl-, Alkenyl- oder Alkoxyrest mit 2 bis 10 C-Atomen, worin auch die direkt mit der Fluoreneinheit verknüpfte $CH_2$-Gruppe durch 1,4-Cyclohexylen oder 1,4-Phenylen ersetzt sein kann,

$R^5$ ist Wasserstoff oder ein geradkettiger Alkyl-, Alkenyl- oder Alkoxyrest mit 2 bis 10 C-Atomen.

**5.** Verwendung von Verbindungen der Formel (I) gemäß einem der Ansprüche 1 bis 4 in Flüssigkristallmischungen.

**6.** Flüssigkristallmischung, enthaltend eine oder mehrere Verbindungen gemäß einem der Ansprüche 1 bis 4 in einer Menge von 1 bis 40 Gew.-%, bezogen auf die Flüssigkristallmischung.

**7.** Flüssigkristallmischung nach Anspruch 6, **dadurch gekennzeichnet, daß** sie nematisch oder smektisch ist.

**8.** Flüssigkristallmischung nach einem der Ansprüche 6 oder 7, enthaltend neben der/den Verbindung(en) der Formel (I) mindestens drei weitere Komponenten.

**9.** Flüssigkristalldisplay, enthaltend eine Flüssigkristallmischung gemäß einem der Ansprüche 6 bis 8.

**10.** Flüssigkristalldisplay nach Anspruch 9, **dadurch gekennzeichnet, daß** es Aktivmatrix-Elemente enthält.

## Claims

**1.** A compound of the formula (I)

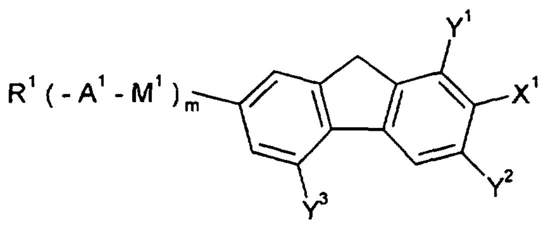

(I)

where
$Y^1$, $Y^2$, $Y^3$ are the same or different and are each H or F with the proviso that at least one of $Y^1$, $Y^2$ is F,
$X^1$ is F, Cl, CN, $CF_3$, $CHF_2$, $CH_2F$, $OCF_3$, $OCHF_2$, $OCH_2F$, $OCH_2CF_3$, $OCH{=}CF_2$ or the group $(M^2 - A^2 -)_n X^2$,
$X^2$ is F, Cl, CN, $CF_3$, $CHF_2$, $CH_2F$, $OCF_3$, $OCHF_2$, $OCH_2F$, $OCH_2CF_3$, $OCH{=}CF_2$ or the group $R^2$,
$R^1$ and $R^2$ are the same or different and are each
hydrogen or a straight-chain or branched alkyl radical having from 1 to 16 carbon atoms or a straight-chain or branched alkenyl radical having from 2 to 16 carbon atoms where

a) one or more nonadjacent and nonterminal $CH_2$ groups may be replaced by -O-, -C(=O)O-, -O-C(=O)-, -O-C(=O)-O-, -C(=O)- or $-Si(CH_3)_2-$, and/or
b) one $CH_2$ group may be replaced by -C≡C-, cyclopropane-1,2-diyl or cyclobutane-1,3-diyl, and/or
c) one or more hydrogen atoms may be replaced by F or Cl, and/or
d) the terminal $CH_3$ group may be replaced by one of the following chiral groups (optically active or racemic):

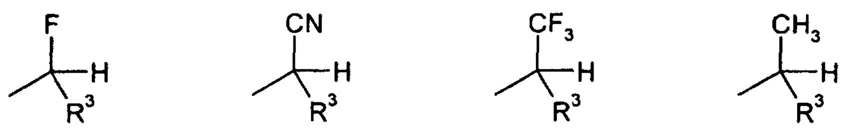

where $R^3$ is a straight-chain or branched alkyl radical having from 1 to 12 carbon atoms or a straight-chain

or branched alkenyl radical having from 2 to 12 carbon atoms, in each of which one or more nonadjacent and nonterminal $CH_2$ groups may be replaced by -O- and/or one or more hydrogen atoms may be replaced by F or Cl, with the proviso that only one of $R^1$ and $R^2$ may be hydrogen,
$M^1$ and $M^2$ are the same or different and are each
-C(=O)-O-, -O-C(=O)-, $-CH_2-O-$, $-O-CH_2-$, $-CF_2-O-$, $-O-CF_2-$, -CH=CH-, -CF=CF-, -C≡C-, $-CH_2-CH_2-C(=O)-O-$, $-O-C(=O)-CH_2-CH_2-$, $-CH_2-CH_2-$, $-CF_2-CF_2-$, $-(CH_2)_4-$ or a single bond,
$A^1$ and $A^2$ are the same or different and are each
1,4-phenylene where one or two hydrogen atoms may be replaced by F, Cl or CN, pyridine-2,5-diyl where one hydrogen atom may be replaced by F, pyrimidine-2,5-diyl, 1,4-cyclohexylene where one or two hydrogen atoms may be replaced by $CH_3$ or F, 1-cyclohexene-1,4-diyl where one hydrogen atom may be replaced by $CH_3$ or F, or 1,3-dioxane-2,5-diyl,
m and n are each independently 0 or 1, but in total not greater than 1.

2. A compound as claimed in claim 1, wherein, in formula (I),
$X^1$ is F, $OCF_3$ or the group $(M^2 - A^2 -)_n X^2$,
$X^2$ is F, $OCF_3$ or the group $R^2$,
$R^1$ and $R^2$ are the same or different and are each
a straight-chain or branched alkyl radical having from 1 to 14 carbon atoms or a straight-chain or branched alkenyl radical having from 2 to 14 carbon atoms, in each of which one or more nonadjacent and nonterminal $CH_2$ groups may be replaced by -O- and/or one or more hydrogen atoms may be replaced by F,
$R^3$ is a straight-chain or branched alkyl radical having from 1 to 10 carbon atoms where one nonterminal $CH_2$ group may be replaced by -O-,
$M^1$ and $M^2$ are the same or different and are each
$-CH_2-O-$, $-O-CH_2-$, $-CH_2-CH_2-$ or a single bond,
$A^1$ and $A^2$ are the same or different and are each
1,4-phenylene where one or two hydrogen atoms may be replaced by F, or 1,4-cyclohexylene, and
m and n are each independently 0 or 1, but in total not greater than 1.

3. A compound as claimed in claim 1, selected from the group of compounds of the formulae (I a) to (I k)

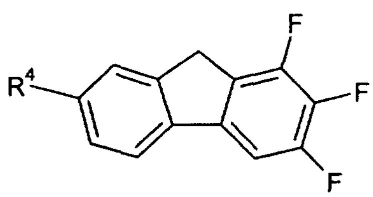

(I a)

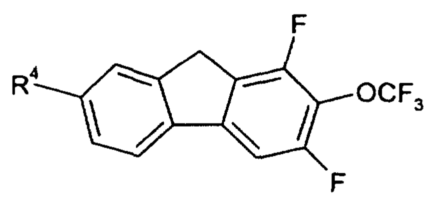

(I b)

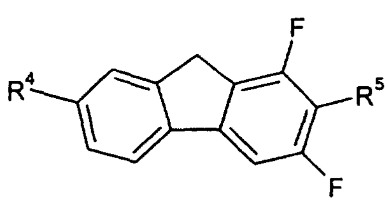

(I c)

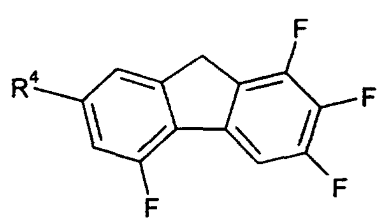

(I d)

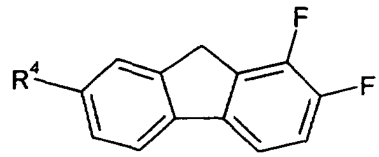

(I e)

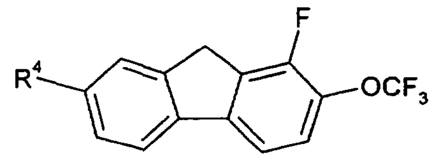

(I f)

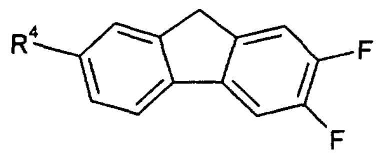

(I g)

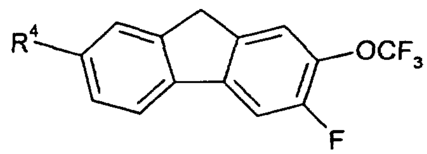

(I h)

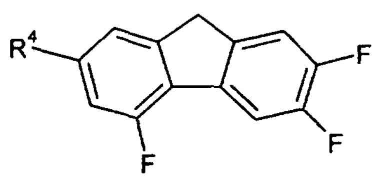

(I i)

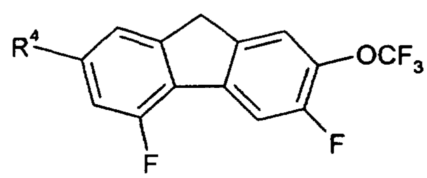

(I k)

where
$R^4$ and $R^5$ are the same or different and are each a straight-chain alkyl, alkenyl or alkoxy radical having from 2 to 12 carbon atoms, wherein the $CH_2$ group directly bound to the fluorene group may be replaced by 1,4-cyclohexylene or 1,4-phenylene which is optionally substituted by one or two F,
$R^5$ is H or a straight-chain alkyl, alkenyl or alkoxy radical having from 2 to 12 carbon atoms, wherein the $CH_2$ group directly bound to the fluorene group may be replaced by 1,4-cyclohexylene or 1,4-phenylene which is optionally substituted by one or two F,
with the proviso that only in one of $R^4$, $R^5$ the $CH_2$ group directly bound to the fluorene group may be replaced by 1,4-cyclohexylene or 1,4-phenylene, which is optionally substituted by one or two F.

**4.** A compound as claimed in claim 3, selected from the group of compounds of the formulae (I a) to (I f), where
$R^4$ is a straight-chain alkyl, alkenyl or alkoxy radical having from 2 to 10 carbon atoms, wherein the $CH_2$ group directly bound to the fluorene group may be replaced by 1,4-cyclohexylene or 1,4-phenylene,
$R^5$ is a straight-chain alkyl, alkenyl or alkoxy radical having from 2 to 10 carbon atoms.

**5.** The use of compounds of the formula (I) as claimed in any of claims 1 to 4 in liquid-crystal mixtures.

**6.** A liquid-crystal mixture comprising one or more compounds as claimed in any of claims 1 to 4 in an amount of from 1 to 40% by weight, based on the liquid-crystal mixture.

**7.** The liquid-crystal mixture as claimed in claim 6, which is nematic or smectic.

**8.** The liquid-crystal mixture as claimed in either of claims 6 or 7, comprising at least three further components in addition to the compound or compounds of the formula (I).

**9.** A liquid-crystal display comprising a liquid-crystal mixture as claimed in any of claims 6 to 8.

**10.** The liquid-crystal display as claimed in claim 9, which comprises active matrix elements.

**Revendications**

**1.** Composé de formule (I)

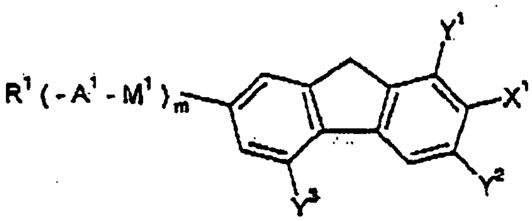

(I)

avec les significations suivantes :

$Y^1$, $Y^2$, $Y^3$ identiques ou différents représentent un atome d'hydrogène ou F, à condition qu'au moins un de $Y^1$, $Y^2$ représente F,

$X^1$ représente F, Cl, CN, $CF_3$, $CHF_2$, $CH_2F$, $OCF_3$, $OCHF_2$, $OCH_2F$, $OCH_2CF_3$, $OCH{=}CF_2$ ou le groupement $(M^2\text{-}A^2\text{-})_nX^2$,

$X^2$ représente F, Cl, CN, $CF_3$, $CHF_2$, $CH_2F$, $OCF_3$, $OCHF_2$, $OCH_2F$, $OCH_2CF_3$, $OCH{=}CF_2$ ou le groupement $R^2$,

$R^1$, $R^2$ identiques ou différents représentent un atome d'hydrogène ou un reste alkyle linéaire ou ramifié présentant 1 à 16 atomes de carbone ou un reste alcényle linéaire ou ramifié présentant 2 à 16 atomes de carbone, où

a) un ou plusieurs groupes $CH_2$ non adjacents et non terminaux peuvent être remplacés par -O-, -C(=O)O-, -O-C(=O)-, -O-C(=O)-O-, -C(=O)- ou $-Si(CH_3)_2-$, et/ou
b) un groupe $CH_2$ peut être remplacé par -C≡C-, cyclopropane-1,2-diyle ou cyclobutane-1,3-diyle, et/ou
c) un ou plusieurs atomes d'hydrogène peuvent être remplacés par un atome de F ou Cl, et/ou
d) le groupe $CH_3$ terminal peut être remplacé par un des groupes chiraux (optiquement actifs ou racémiques) suivants :

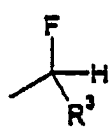 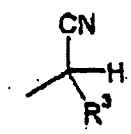 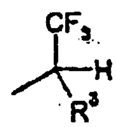 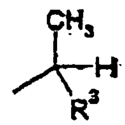

$R^3$ représente un reste alkyle linéaire ou ramifié présentant 1 à 12 atomes de carbone ou un reste alcényle linéaire ou ramifié présentant 2 à 12 atomes de carbone, où un ou plusieurs groupes $CH_2$ non adjacents et non terminaux peuvent être remplacés par un -O- et/ou un ou plusieurs atomes d'hydrogène peuvent être remplacés par F ou Cl,

à condition que seulement un de $R^1$, $R^2$ puisse représenter un atome d'hydrogène,

$M^1$, $M^2$ identiques ou différents représentent un groupe -C(=O)O-, -O-C(=O)-, $-CH_2O-$, $-O-CH_2-$, $-CF_2O-$, $-O-CF_2-$, -CH=CH-, -CF=CF-, -C≡C-, $-CH_2-CH_2-C(=O)-O-$, $-O-C(=O)-CH_2-CH_2-$, $-CH_2-CH_2-$, $-CF_2-CF_2-$, $-(CH_2)_4-$ ou une liaison simple,

$A^1$, $A^2$ identiques ou différents représentent un groupe 1,4-phénylène, où un ou deux atomes d'hydrogène peuvent être remplacés par F, Cl ou CN, pyridine-2,5-diyle, où un atome d'hydrogène peut être remplacé par F, pyrimidine-2,5-diyle, 1,4-cydohexylène, où un ou deux atomes d'hydrogène peuvent être remplacés par $CH_3$ ou F, 1-cyclohexène-1,4-diyle, où un atome d'hydrogène peut être remplacé par

$CH_3$ ou F, ou 1,3-dioxane-2,5-diyle,

m, n indépendamment l'un de l'autre valent 0 ou 1, mais dont la somme n'est pas supérieure à 1.

**2.** Composé selon la revendication 1, **caractérisé en ce qu'**à la formule (I) représentent

| | |
|---|---|
| $X^1$ | F, $OCF_3$ ou le groupement $(M^2\text{-}A^2\text{-})_nX^2$, |
| $X^2$ | F, $OCF_3$ ou le groupement $R^2$, |
| $R^1$, $R^2$ | identiques ou différents représentent un reste alkyle linéaire ou ramifié présentant 1 à 14 atomes de carbone ou un reste alcényle linéaire ou ramifié présentant 2 à 14 atomes de carbone, où un ou plusieurs groupes $CH_2$ non adjacents et non terminaux peuvent être remplacés par un -O- et/ou un ou plusieurs atomes d'hydrogène peuvent être remplacés par F, |
| $R^3$ | représente un reste alkyle linéaire ou ramifié présentant 1 à 10 atomes de carbone où un groupe $CH_2$ non terminal peut être remplacé par un -O-, |
| $M^1$, $M^2$ | identiques ou différents représentent un groupe $-CH_2O-$, $-O-CH_2-$, $-CH_2-CH_2-$ ou une liaison simple, |
| $A^1$, $A^2$ | identiques ou différents représentent un groupe 1,4-phénylène, où un ou deux atomes d'hydrogène peuvent être remplacés par F ou représentent 1,4-cydohexylène, |
| m, n | indépendamment l'un de l'autre valent 0 ou 1, mais dont la somme n'est pas supérieure à 1. |

**3.** Composé selon la revendication 1, pris dans le groupe de composés de formules (I a) à (I k)

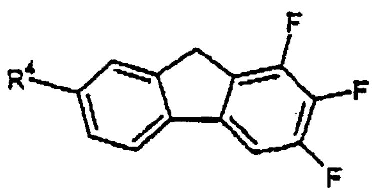

(I a)

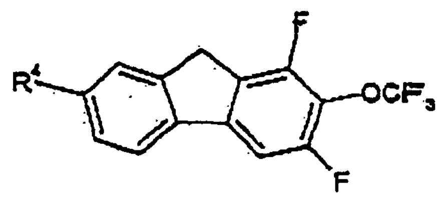

(I b)

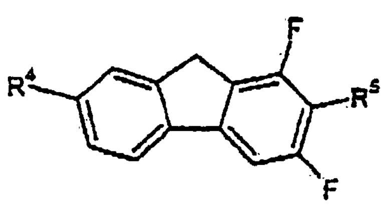

(I c)

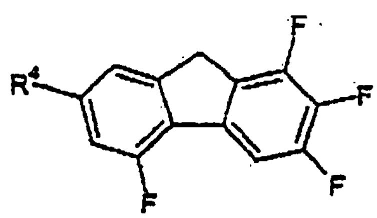

(I d)

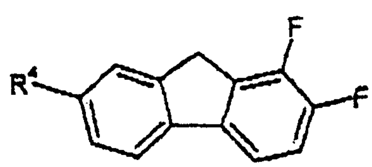

(I e)

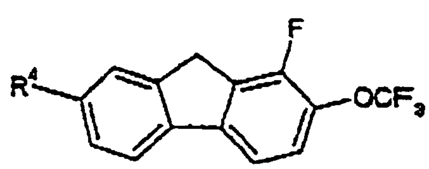

(I f)

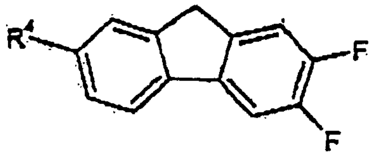

(I g)

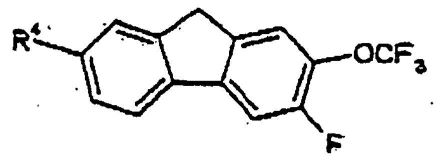

(I h)

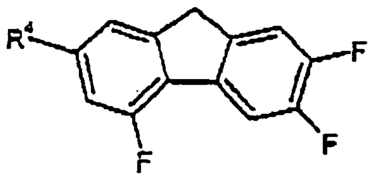

(I i)

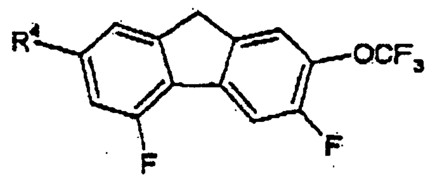

(I k)

avec les significations :

$R^4$ représente un reste alkyle, alcényle ou alkoxy linéaire présentant 2 à 12 atomes de carbone, dans lesquels aussi le groupe $CH_2$ lié directement à l'unité fluor peut être remplacé par un groupe 1,4-cyclohexylène ou 1,4-phénylène, qui est éventuellement substitué par un ou deux F,

$R^5$ représente un atome d'hydrogène ou un reste alkyle, alcényle ou alkoxy linéaire présentant 2 à 12 atomes de carbone, dans lesquels aussi le groupe $CH_2$ lié directement à l'unité fluor peut être remplacé par un groupe 1,4-cyclohexylène ou 1,4-phénylène, qui est éventuellement substitué par un ou deux F, à condition que seulement dans un de $R^4$, $R^5$ le groupe $CH_2$ lié directement à l'unité fluor puisse être remplacé par un groupe 1,4-cyclohexylène ou 1,4-phénylène, qui est éventuellement substitué par un ou deux F.

**4.** Composés selon la revendication 3, pris dans le groupe des composés de formules (I a) à (I f), où :

$R^4$ représente un reste alkyle, alcényle ou alkoxy linéaire présentant 2 à 10 atomes de carbone, dans lesquels aussi le groupe $CH_2$ lié directement à l'unité fluor peut être remplacé par un groupe 1,4-cyclohexylène ou 1,4-phénylène,

$R^5$ représente un atome d'hydrogène ou un reste alkyle, alcényle ou alkoxy linéaire présentant 2 à 10 atomes de carbone.

**5.** Utilisation de composés de formule (I) selon l'une des revendications 1 à 4 dans des mélanges de cristaux liquides.

**6.** Mélange de cristaux liquides renfermant un ou plusieurs composés selon l'une des revendications 1 à 4 en une quantité de 1 à 40 % en masse, par rapport au mélange de cristaux liquides.

**7.** Mélange de cristaux liquides selon la revendication 6, **caractérisé en ce qu'**il est nématique ou smectique.

**8.** Mélange de cristaux liquides selon l'une des revendications 6 ou 7, renfermant au côté du/des composé(s) de formule (I) au moins trois autres composants.

**9.** Afficheur à cristal liquide renfermant un mélange de cristaux liquides selon l'une des revendications 6 à 8.

**10.** Afficheur à cristal liquide selon la revendication 9, **caractérisé en ce qu'**il renferme des éléments de matrice active.